# EUROPEAN PATENT SPECIFICATION

(11) **EP 4 013 374 B1**
(45) Date of publication and mention of the grant of the patent: **19.02.2025**
(21) Application number: 20853893.4
(22) Date of filing: 18.08.2020
(51) Int. Cl.: C08B 37/02, C08B 37/16, C08B 37/08, A61K 8/49, A61Q 17/04, C08B 15/00

(54) **COMPOSITIONS FOR SUNSCREEN COMPOUNDS AND METHODS THEREOF**
ZUSAMMENSETZUNGEN FÜR SONNENSCHUTZVERBINDUNGEN UND VERFAHREN DAFÜR
COMPOSITIONS DE COMPOSÉS D'ÉCRAN SOLAIRE ET PROCÉDÉS CORRESPONDANTS

(30) Priority: 18.08.2019 US 201962888526 P; 04.10.2019 US 201962910902 P
(43) Date of publication of application: 22.06.2022
(60) Divisional application: 24223533.1
(73) Proprietor: PMIDG, LLC, Anderson, South Carolina 29625 (US)
(72) Inventor: GRAVETT, David, Anderson, South Carolina 29625 (US)
(74) Representative: Uexküll & Stolberg
(86) International application number: PCT/US2020/046811
(87) International publication number: WO 2021/034834

(56) References cited:
- US-A- 4 524 061
- US-A1- 2006 067 893
- US-A1- 2015 150 827
- US-A1- 2018 092 819
- US-B1- 6 214 324
- TROMMER ET AL.: "Screening For New Antioxidative Compounds For Topical Administration Using Skin Lipid Model Systems", J PHARM PHARMACEUT SCI, vol. 8, no. 3, 15 September 2005 (2005-09-15), pages 494 - 506, XP009092520

## Description

### TECHNICAL FIELD

The present disclosure relates to compositions comprising compounds for preventing absorption of UV radiation, particularly for use in topical applications.

### BACKGROUND

More humans are using sunscreen compositions, such as those that reflect ultra-violet (UV) radiation away from exterior surfaces of the body, in greater frequency and for longer periods than was previously known. Humans are applying sunscreens daily, and are using such compositions on all ages, from infants to the elderly. Previously, humans applied sunscreen for outdoor activities on a limited basis.

The longer period of use, by more people, in greater frequency and for years, has led to questions regarding the safety of sunscreen compositions. It was long assumed that the UV-reflective compounds were not absorbed through the skin into the general circulation of the body. But recent studies have shown that such compounds are absorbed by the skin and enter the interior of the body. For example, in 2019, in the Journal of the American Medical Association, Matta et al, describe the results of an exploratory maximal usage trial (MUsT) that studied the systemic absorption (through the skin and into the body) of sunscreen active ingredients using four commercially available sunscreen products. A MUsT study evaluates the systemic absorption of a topical drug (i.e., one applied to the skin) when used according to the maximum limits of the product's directions for use. In this pilot study, all four active ingredients tested were absorbed from each formulation tested, showing that absorption of sunscreens is not just a theoretical concern. The U.S. Food and Drug Administration (FDA) has issued a proposed rule to update regulatory requirements for most sunscreen products in the United States, where sunscreens are regulated as drugs. As part of this rule, the FDA is requesting additional safety data on 12 active sunscreen ingredients currently available in marketed products because there is no data regarding whether, and to what extent, the ingredient is absorbed into the body after topical application.

Even with this increased use of sunscreens by humans, the incidence rates of skin cancer continue to rise, with skin cancer remaining the most commonly diagnosed cancer in the United States. This incidence rate makes risk from excess sun exposure an important public health priority. Broad spectrum sunscreens with SPF values of at least 15 are needed for preventing skin cancer and protecting the skin from sunburn and other UV damage. What is needed are formulation compositions, such as sunscreen formulations, comprising at least one UV absorbing conjugate compound, that are not absorbed, transmitted and/or transported across a body surface barrier, or are poorly absorbed, transmitted and/or transported or are slowly absorbed, transmitted and/or transported across body surfaces, such as skin, hair, nails, and mucous membranes, and that do not readily enter the general circulation of the body.

US2018092819 describes UV radiation absorbing polymer composition, useful in a cosmetic composition, and as sunscreen active for protection of human skin against action of UV radiation.

### SUMMARY

The present disclosure comprises compositions comprising at least one UV absorbing conjugate compound that reflects and/or absorbs UV radiation, such as UV-A, UV-B and/or both UV-A and UV-B, that are not absorbed by, transmitted across and/or transported from the surface of the subject's body to the interior of the body or circulatory system of the subject's body. In an aspect, disclosed herein are formulation compositions comprising a benzotriazole compound conjugated to a conjugate compound via an amide linker wherein the benzotriazole conjugate compound has a molecular weight of at least 800 daltons and wherein, the benzatriazole compound absorbs ultraviolet light; formulation compositions comprising a benzotriazole compound conjugated to conjugate compound through the product of a Michael addition reaction wherein the benzotriazole conjugate compound has a molecular weight of at least 800 daltons and wherein, the benzatriazole compound absorbs ultraviolet light; formulation compositions comprising a benzotriazole functionalized polymer wherein benzotriazole functionalized polymer has a molecular weight of at least 800 daltons and wherein the benzotriazole is bonded to the polymer as a methacrylate, acrylate or acrylaminde derivative of a benzotriazole compound and wherein, the benzotriazole compound absorbs ultraviolet light; and/or formulation compositions comprising a benzotriazole compound conjugated to a polysaccharide wherein the benzotriazole polysaccharide conjugate compound has a molecular weight of at least 800 daltons and wherein, the benzatriazole compound absorbs ultraviolet light. In an aspect, a UV absorbing conjugate compound (UV absorbing compound) comprises the structure shown in Formula (1), (U -X)ₙ -C, wherein, U is an UV absorbing compound moiety, X is a thioether, amine, amide, ester, urethane, sulfonamide group, or -CO-NH-NH-CO-, C is a conjugate compound moiety and n is an integer and where n is ≥1.

In an aspect, an UV absorbing conjugate compound has the structure shown in Formula (2), 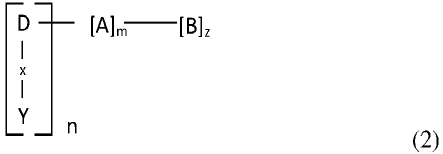 wherein Y is an UV absorbing compound moiety, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n is an integer and where n is ≥1 and m and z are each an integer and where m is ≥0, and z is ≥0. The structure in Formula (2) can be a block copolymer or it can be a random copolymer.

In an aspect, a UV absorbing conjugate compound has the structure shown in Formula (3),

(U -X)n -C-(X-U1)m (3)

wherein, U is an UV absorbing compound moiety, U1 is a UV absorbing compound moiety that has a different chemical structure to U, X is a thioether, amine, amide, ester, urethane, sulfonamide group, -CO-NH-NH-CO-, C is a conjugate compound moiety and n and m are each an integer and where n and m is ≥1.

In an aspect, , an UV absorbing conjugate compound has the structure shown in Formula (4), 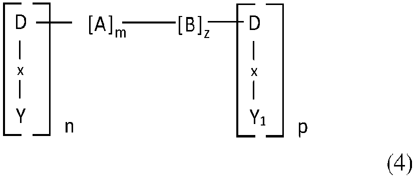 wherein Y is an UV absorbing compound moiety, Y₁ is a UV absorbing compound moiety that has a different chemical structure to Y, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n and p are each an integer and where n and p is ≥1 and m and z are each an integer and where m is ≥0, and z is ≥0. The structure in Formula (4) can be a block copolymer or it can be a random copolymer.

### FIGURES

FIG.1 shows exemplary UV absorbing conjugate compounds comprising benzotriazole acrylate UV absorbing compounds and small molecules as the conjugate compound.

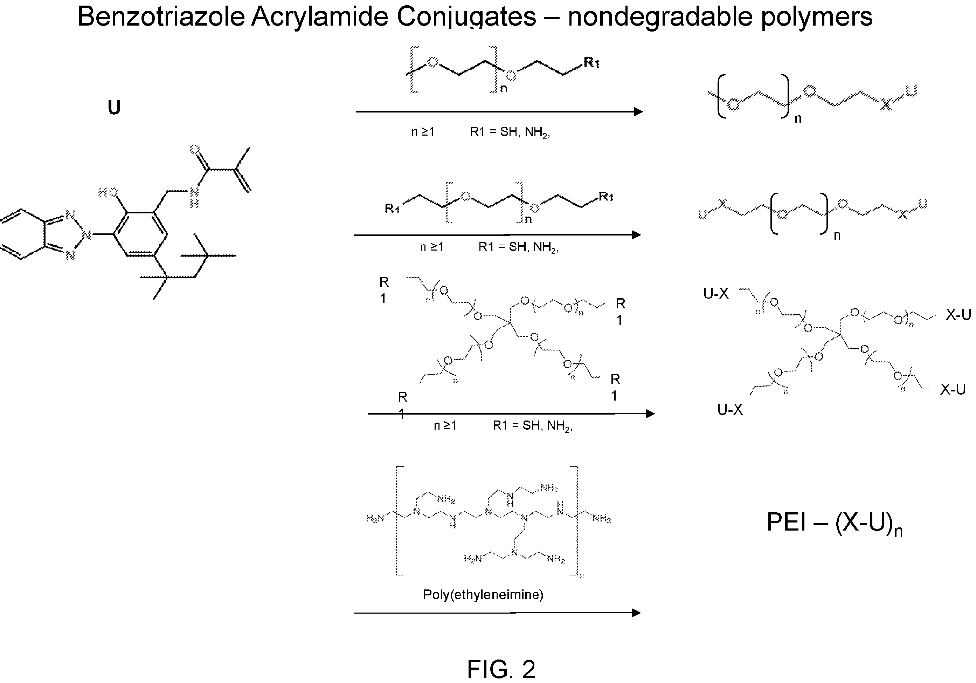

FIG.2 shows exemplary UV absorbing conjugate compound comprising benzotriazole acrylamide UV absorbing compound and non-degradable polymeric molecules as the conjugate compound.

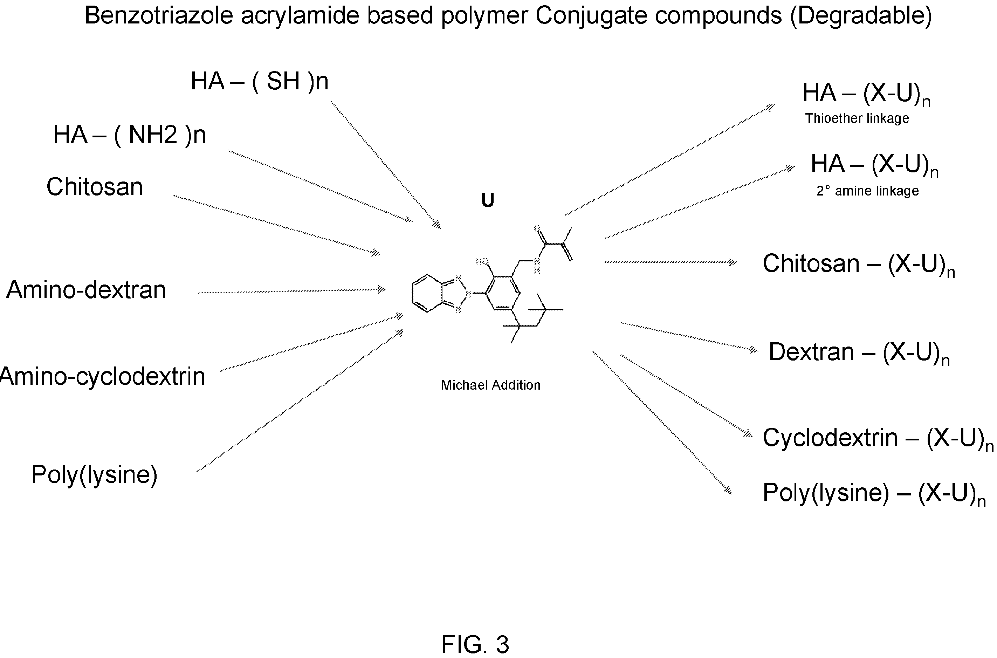

FIG.3 shows exemplary UV absorbing conjugate compounds comprising benzotriazole acrylamide UV absorbing compound and degradable polymeric molecules as the conjugate compound.

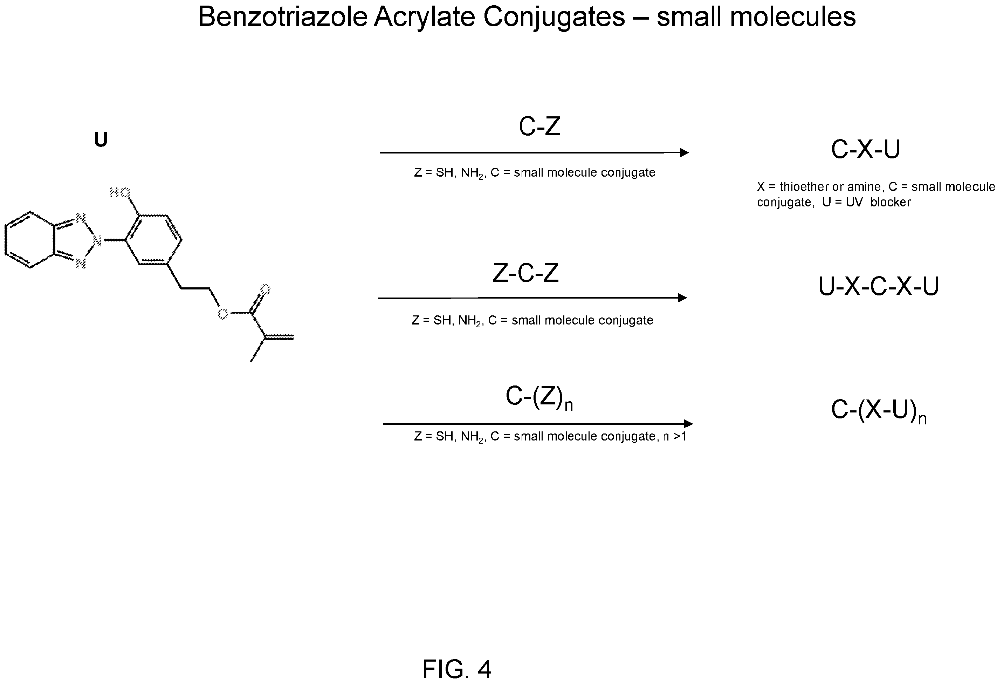

FIG.4 shows exemplary UV absorbing conjugate compounds comprising benzotriazole acrylate UV absorbing compound and small molecules as the conjugate compound.

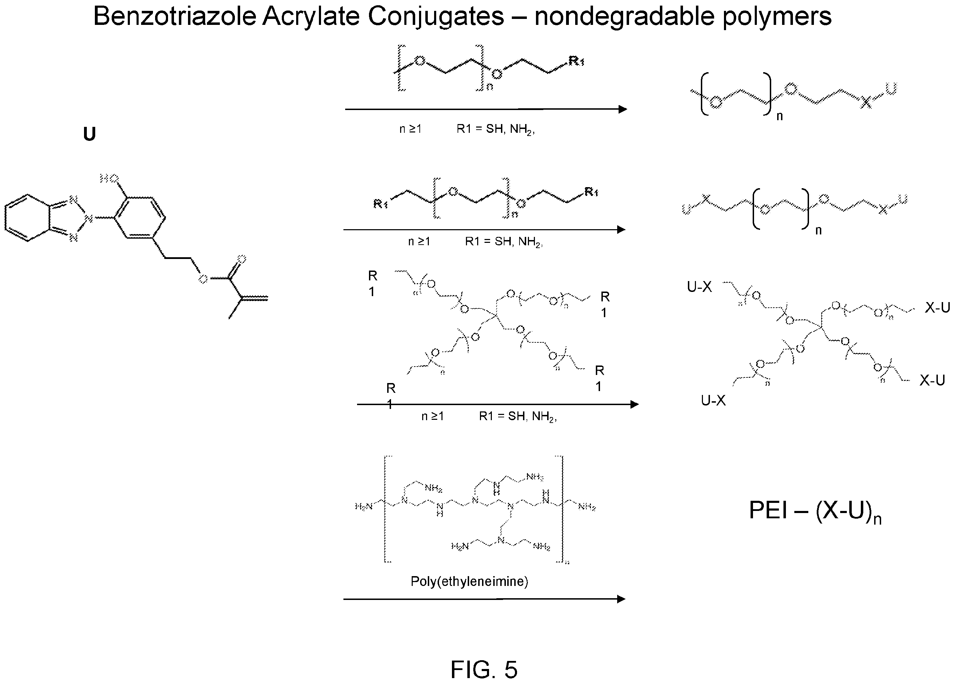

FIG.5 shows exemplary UV absorbing conjugate compounds comprising benzotriazole acrylate UV absorbing compound and polymeric molecules as the conjugate compound.

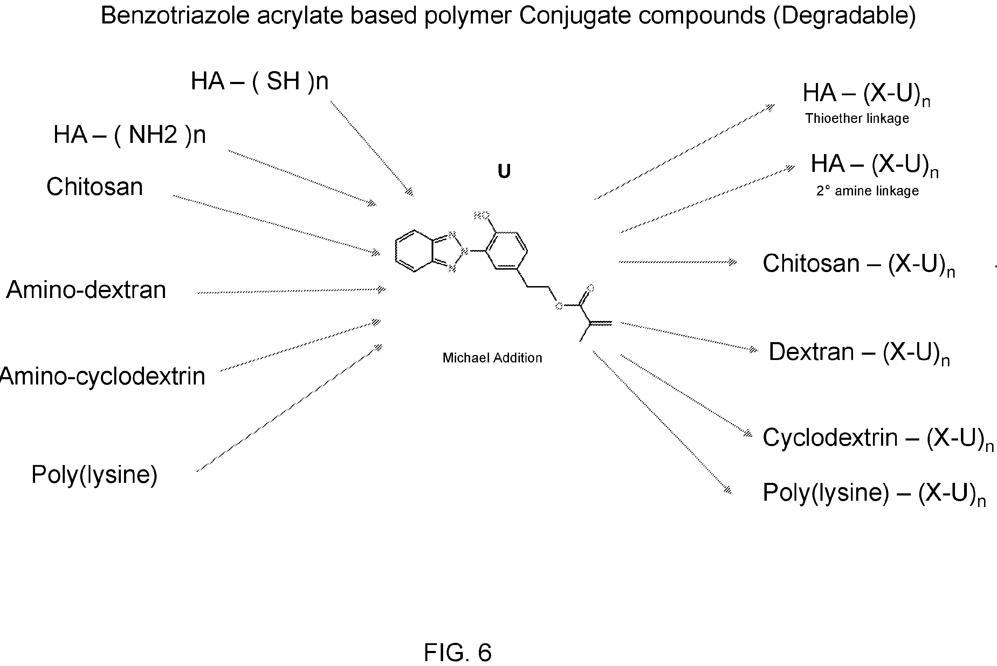

FIG.6 shows exemplary UV absorbing conjugate compounds comprising benzotriazole acrylate UV absorbing compound and absorbable/degradable molecules as the conjugate compound.

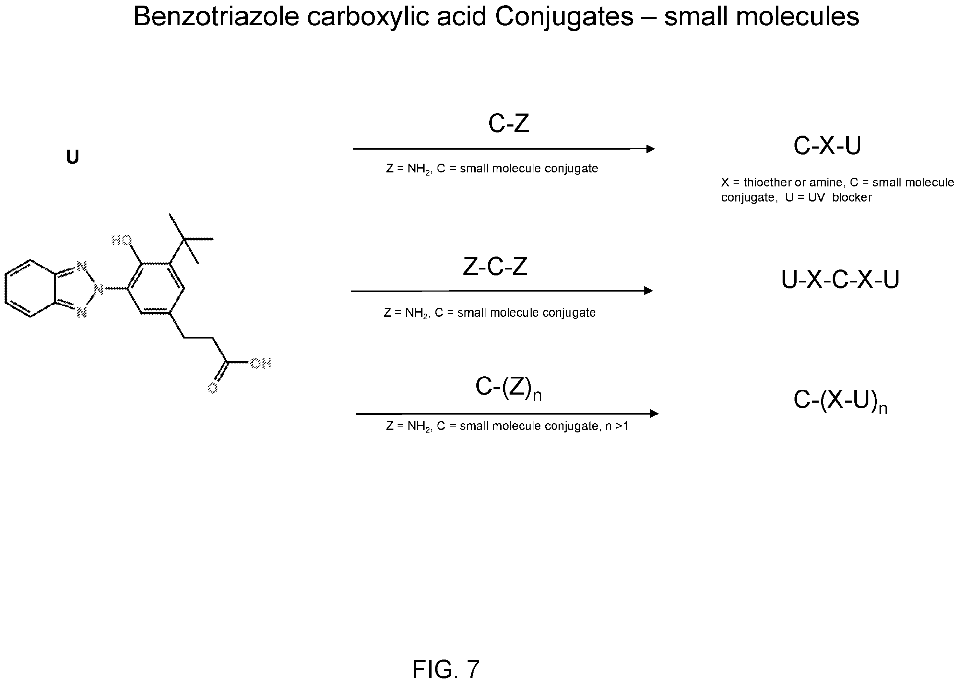

FIG.7 shows exemplary UV absorbing conjugate compounds comprising benzotriazole carboxylate UV absorbing compounds and small molecules as the conjugate compound.
FIG.8 shows exemplary UV absorbing conjugate compound comprising benzotriazole carboxylate UV absorbing compound and non-degradable polymeric molecules as the conjugate compound.
FIG.9 shows exemplary UV absorbing conjugate compounds comprising benzotriazole carboxylate UV absorbing compound and degradable polymeric molecules as the conjugate compound.
FIG.10 shows exemplary UV absorbing conjugate compounds comprising linear polymers comprising benzotriazole UV absorbing compounds.

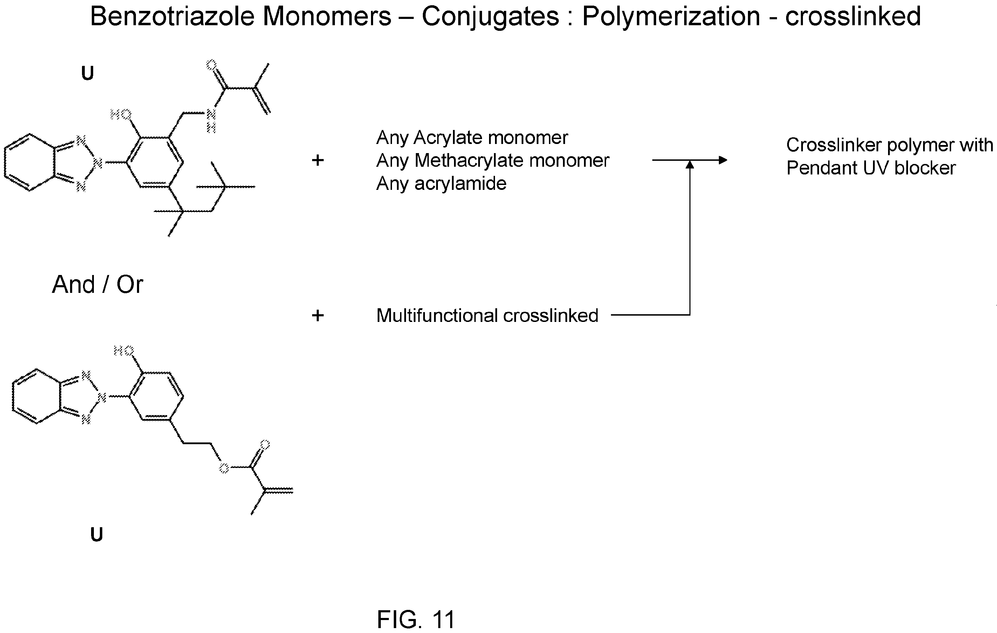

FIG.11 shows exemplary UV absorbing conjugate compound comprising crosslinked polymers comprising benzotriazole UV absorbing compounds.
FIG.12 shows exemplary UV absorbing conjugate compounds comprising UV absorbing compound comprising sulfonate and small molecules as the conjugate compound as the conjugate compound.

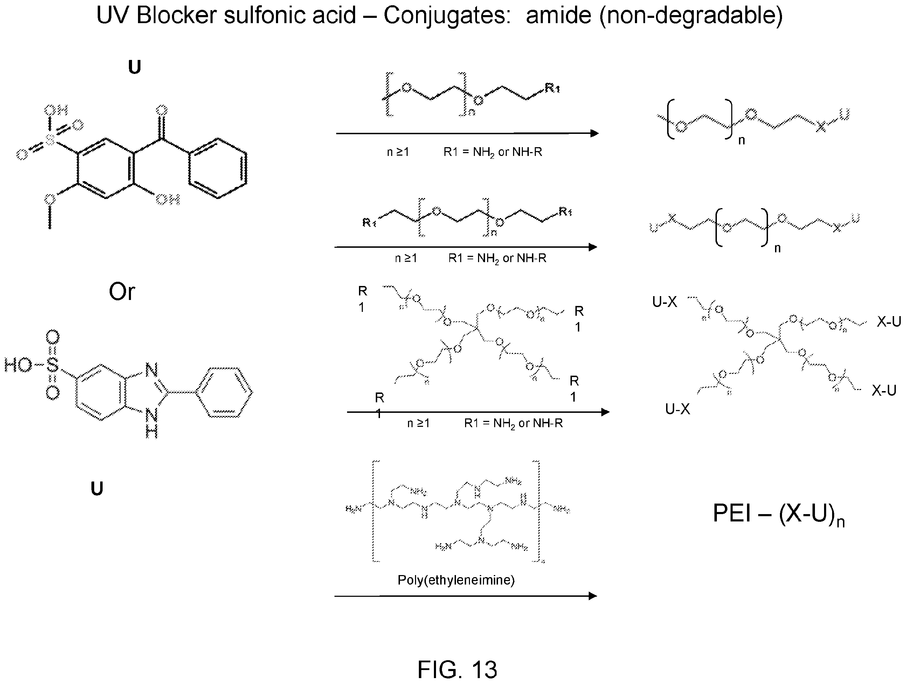

FIG.13 shows exemplary UV absorbing conjugate compound comprising UV absorbing compound comprising sulfonic acid and non-degradable polymeric molecules as the conjugate compound.

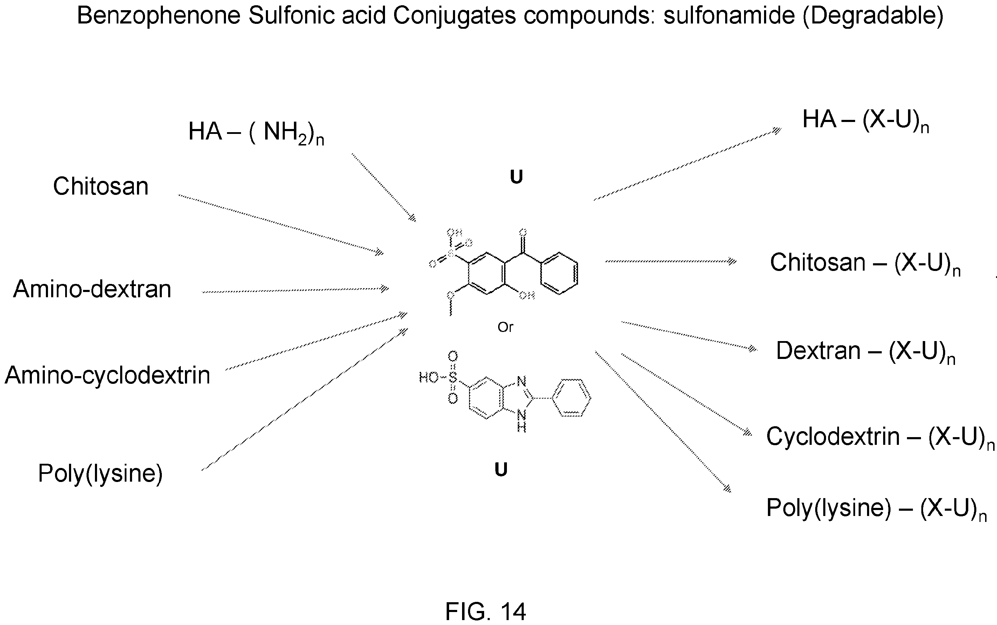

FIG.14 shows exemplary UV absorbing conjugate compounds comprising UV absorbing compound comprising sulfonic acid and degradable polymeric molecules as the conjugate compound.
FIG.15 shows exemplary UV absorbing conjugate compounds comprising benzotriazole hydrazide UV absorbing compounds and small molecules as the conjugate compound.

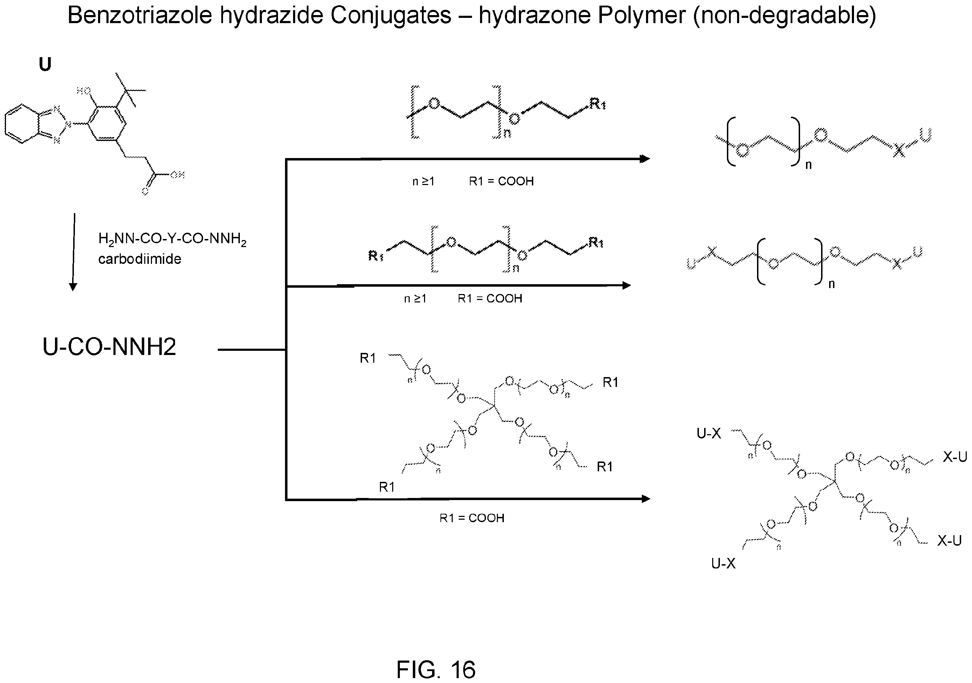

FIG.16 shows exemplary UV absorbing conjugate compound comprising benzotriazole hydrazide UV absorbing compound and non-degradable polymeric molecules as the conjugate compound.
FIG.17 shows exemplary UV absorbing conjugate compounds comprising benzotriazole hydrazide UV absorbing compound and degradable polymeric molecules as the conjugate compound.
FIG. 18 is a graph of NMR results of the UV absorbing conjugate compound of Example 16.
FIG. 19 is a graph of the UV spectrum of the UV absorbing conjugate compound in dichloromethane of Example 16.
FIG. 20 is a graph of NMR results of the UV absorbing conjugate compound of Example 18.
FIG. 21 is a graph of the UV spectrum of the UV absorbing conjugate compound in dichloromethane of Example 18.

### DETAILED DESCRIPTION

Disclosed herein are compositions comprising at least one UV absorbing conjugate compound and methods for making and using such compositions. As used herein, an UV absorbing compound is a compound that reflects and/or absorbs radiation in certain ultraviolet (UV) wavelengths. In an aspect, disclosed herein are inorganic compounds that reflect or scatter the light away from the skin, and organic (carbon-based) compounds that absorb UV rays. Some inorganic compounds, including zinc oxide or titanium dioxide, act as a physical sunblock preventing radiation from reaching the body surface.

Examples of organic compounds that are UV absorbing compounds include, but are not limited to avobenzone or oxybenzone. Instead of physically deflecting UV rays, these molecules absorb UV radiation through their chemical bonds. As the bonds absorb UV radiation, the components of the sunscreen slowly break down and release heat.

SPF is often used to show the protective level of a sunscreen composition. SPF is Sun Protection Factor, and refers to how well the sunscreen protects against UVB rays, which can cause sunburn and several types of skin cancer. UVA radiation, penetrates deeper into the skin and can cause premature wrinkling, age spots and can also heighten the risk for some skin cancers. A broad spectrum sunscreen blocks against both UVA and UVB rays, but currently there is no standard for listing UVA absorbing power. Inorganic compounds that deflect sunlight will deflect both UVA and UVB rays.

It is recommended to use a sunscreen composition that is rated as SPF 15 to 50, as it is unproven that those rated higher than SPF 50 are any more effective that SPF 50. A sunscreen with an SPF of 15 protects against about 93 percent of UVB rays, and one with an SPF of 30 protects against 97 percent of rays, though no composition can block 100 percent of UV rays. A sunscreen composition will not eliminate UV radiation from reaching the body's surface, and thus, the SPF number refers to approximately the length of time it will take for a person's skin to show radiation damage, such as turning red. A sunscreen composition with an SPF of 15 will prevent Caucasian skin from getting red for approximately 15 times longer than skin that is not coated with the sunscreen composition. For example, if the skin starts to burn (turning red) in 10 minutes, a sunscreen composition with SPF 15 will prevent burning for about 150 minutes, or 2.5 hours.

Disclosed herein are formulation compositions comprising at least one UV absorbing conjugate compound composition, for example, cosmetic compositions, comprising one or more compounds or molecules that reflect and/or absorb UV-A, UV-B or both UV-A and UV-B radiation, for use in preventing or ameliorating the harmful effects of UV radiation, such as that from the sun. It is desirable that the UV absorbing conjugate compounds are not significantly absorbed or transported through the body surface, such as skin, into the general circulation of the body. The biological activity and toxicity of many of the currently used UV absorbing and or UV reflecting compounds have not been studied.

Disclosed herein are UV absorbing conjugate compounds comprising a UV absorbing compound conjugated to a conjugate compound, which may comprise a small molecule or a polymer. In an aspect, compositions disclosed herein comprise at least one UV absorbing compound for which its effective molecular weight is increased by conjugating the conjugate compound to the UV absorbing compound in order to reduce the absorption of the UV absorbing compound through the skin. In an aspect, the effective molecular weight of an UV absorbing compound can be increased by covalently coupling the UV absorbing compound to a conjugate compound or molecule such that the total molecular weight of the conjugated molecule (referred to herein as an UV absorbing conjugate compound) is greater than 500 daltons, or greater than 800 daltons, or greater than 1000 daltons. For brevity, herein a UV absorbing conjugated compound may be referred to herein as an "absorbing compound" and includes a compound that absorbs UV radiation, or reflects UV radiation, or both absorbs and reflects UV radiation.

In an aspect, a UV absorbing conjugate compound (UV absorbing compound) has the structure shown in Formula (1),

(U-X)ₙ-C (1)

wherein, U is an UV absorbing compound moiety, X is a thioether, amine, amide, ester, urethane, sulfonamide group, or -CO-NH-NH-CO-, C is a conjugate compound moiety and n is an integer and where n is ≥ 1.

In an aspect, an UV absorbing conjugate compound has the structure shown in Formula (2), 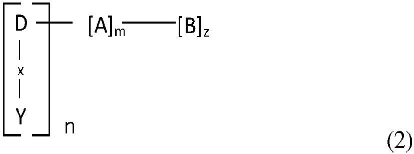 wherein Y is an UV absorbing compound moiety, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n is an integer and where n is ≥1 and m and z are each an integer and where m is ≥0, and z is ≥0. The structure in Formula (2) can be a block copolymer or it can be a random copolymer.

In an aspect, a UV absorbing conjugate compound has the structure shown in Formula (3),

(U-X)ₙ-C-(X-U₁)ₘ (3)

wherein, U is an UV absorbing compound moiety, U₁ is a UV absorbing compound moiety that has a different chemical structure to U, X is a thioether, amine, amide, ester, urethane, sulfonamide group, -CO-NH-NH-CO-, C is a conjugate compound moiety and n and m are each an integer and where n and m is ≥1.

In an aspect, an UV absorbing conjugate compound has the structure shown in Formula (4), 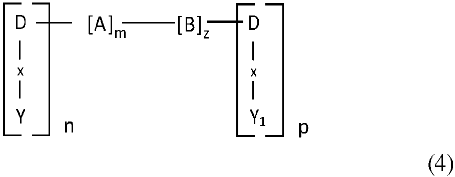 wherein Y is an UV absorbing compound moiety, Y₁ is a UV absorbing compound moiety that has a different chemical structure to Y, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n and p are each an integer and where n and p is ≥1 and m and z are each an integer and where m is ≥0, and z is ≥0. The structure in Formula (4) can be a block copolymer or it can be a random copolymer.

Compositions of the present disclosure may comprise one or more UV absorbing conjugate compounds that comprise a UV absorbing compound conjugated (bonded) with a conjugate compound. In an aspect, a conjugate compound, not including a conjugate compound comprising a polymer (described below), of a UV absorbing conjugate compound is a small molecule with a molecular weight of less than 1000 daltons, in a range from about 100 daltons to about 1000 daltons, from about 100 daltons to about 900 daltons, from about 200 daltons to about 8000 daltons, from about 200 daltons to about 1000 daltons, from about 100 daltons to about 300 daltons, from about 200 daltons to about 500 daltons, from about 300 daltons to about 1000 daltons; from about 400 daltons to about 8000 daltons; from about 400 daltons to about 1000 daltons; from about 500 daltons to about 1000 daltons; from about 400 daltons to about 800 daltons from about 500 daltons to about 600 daltons; from about 600 daltons to about 1000 daltons; from about 600 daltons to about 800 daltons; from about 700 daltons to about 1000 daltons; from about 800 daltons to about 1000 daltons; from about 800 daltons to about 900 daltons; from about 900 daltons to about 1000 daltons, and all ranges therein between. In an aspect, a conjugate compound has one or more functional groups that can react with a functional group of a UV absorbing compound. A functional group may be a component of a conjugate compound or may be added by chemical synthesis methods to a conjugate group. Functional groups on a conjugate compound include, but are not limited to, one or more thiol groups, amine groups, hydrazide group, carboxylic acid groups or a combination thereof. In an aspect, a conjugate compound can be an alkane. In an aspect, a conjugate compound is a linear alkane. In an aspect, a conjugate compound is a branched alkane. In an aspect, a conjugate compound comprises an aromatic ring. In an aspect, a conjugate compound comprises a benzene ring. In an aspect, a conjugate compound comprises two or more thiol or amine groups. In an aspect, a conjugate compound comprises at least one thiol group and at least one amine group. In an aspect, an amine group is a primary amine group. In an aspect, a conjugate compound comprises one or more carboxylic acid groups. In an aspect, a conjugate compound comprises one or more hydrazide groups.

In an aspect, a conjugate compound with two or more amine groups can be reacted with one or more UV absorbing compounds that have a carboxylic acid group, an acrylate group, a methacrylate group or an acrylamide group. For example, a conjugate compound that is an alkane, with two or more amine groups, include, but is not limited to, C2 to C20 diamines, 1,5-Diamino-2-methylpentane, 1,3-Diamino-2-propanol, 3,3'-Diamino-N-methyldipropylamine, 1,3-Diamino-2-hydroxypropane-N,N,N',N'-tetraacetic acid, meso-1,4-Diamino-2,3-butanediol dihydrochloride, 1,4-Diamino-2-butanone dihydrochloride, 2,2- Bis(aminoethoxy)propane, Spermidine, DL-5-Hydroxylysine hydrochloride, triethylenetetramine, tetraethylenepentamine cystine, diethylenetriamine, bis(3-aminopropyl)amine, N,N'-bis(2-aminoethyl)-1,3-propanediamine, 4,9-Dioxa-1,12-dodecanediamine, and 2,6-Diaminopimelic acid.

In an aspect, a conjugate compound that comprise a benzene ring and at least two amine groups includes, but is not limited to, 1,2-diamino-3,5-dimethylbenzene, 4,4'-diamino[1,1'-biphenyl]-3,3'-diol, 1,2-diamino-4,5-dimethoxybenzene, 4,4'-diamino-2,2'-stilbenedisulfonic acid, 6,9-diamino-2-ethoxyacridine-DL-lactate monohydrate, 2,2'-diamino-4,4'-stilbenedicarboxylic acid, 3,8-diamino-6-phenylphenanthridine, 2,6-diamino-4-phenyl-1,3,5-triazine, m-xylylenediamine, p-xylylenediamine, o-xylylenediamine, 2,4,6-Trimethyl-m-phenylenediamine, 2,2,4(2,4,4)-trimethyl-1,6-hexanediamine, 4,4'-methylene-bis(2-methylaniline), 4,4'-methylene-bis(2-chloroaniline), 2,5-dichloro-p-phenylenediamine, 4,4'-dibromo-2,2'-biphenyldiamine, 2,6-diaminotoluene, 2-methyl-m-phenylenediamine, benzidine, 2,4-diaminobenzenesulfonic acid, 2,4-diaminophenol dihydrochloride, 1,4-diaminoanthraquinone, 2,6-diaminoanthraquinone, 1,5-diaminoanthraquinone, 1,2-diaminoanthraquinone, 3,5-diaminobenzoic acid dihydrochloride, and 4,4'-diaminobenzanilide.

In an aspect, a conjugate compound that comprises two or more amine groups include, but is not limited to, 3,5-diamino-1,2,4-triazole, 2,4-diamino-6-hydroxypyrimidine, 5,6-diamino-1,3-dimethyluracil hydrate, 2,4-diamino-6-phenyl-1,3,5-triazine, 2,4-diamino-6-(hydroxymethyl)pteridine, 2,4-diamino-6-(hydroxymethyl)pteridine hydrochloride, 2,6-diamino-3,5-difluoropyridine, 2-chloro-4,6-diamino-1,3,5- triazine, 2-nitro-1,4-phenylenediamine, 2,6-diaminopurine-9-arabinoside, melamine, 6-methyl-1,3,5-triazine-2,4-diamine, 2,6-diaminopyridine, 2,5-diaminopyridine, 2,4-diaminopyrimidine, lysine, esters of lysine such as lysine methyl ester, lysine ethyl ester, arginine, esters of arginine such as arginine methyl ester, and histidine.

In an aspect, a conjugate compound with two or more thiol groups can be reacted with one or more UV absorbing compounds having an alkene group to form a UV absorbing conjugate compound comprising a thioether. Alkene groups include, but are not limited to, a vinyl group, an acrylate group, a methacrylate group, a cyanoacrylate group or an acrylamide group. Conjugate compounds that are alkanes with two or more thiol groups include, but are not limited to, C2 to C20 dithiols, dithiothreitol, and 2,3-dimercapto-1-propanol, and 2,3-dimercaptosuccinic acid.

In an aspect, a conjugate compound comprising a benzene ring and at least two thiol groups includes, but is not limited to, 1,4-benzenedimethanethiol, 1,4-benzenedimethanethiol, benzene-1,2-dithiol, benzene-1,4-dithiol, 1,3-benzenedithiol, biphenyl-4,4'-dithiol, and p-terphenyl-4,4"-dithiol.

In an aspect, a conjugate compound comprising at least one thiol and one amine group include, but are not limited to, 2-aminobutane-1,4-dithiol hydrochloride, 4,5-diamino-2,6-dimercaptopyrimidine, 4,5-diamino-6-hydroxy-2-mercaptopyrimidine, and 4,6-diamino-2-pyrimidinethiol.

In an aspect, a conjugate compound comprising two or more hydrazide are comprised by the present disclosure. One or more hydrazide groups can be reacted with a carboxylic acid group of the UV absorbing compound. Dihydrazide compounds include, but are not limited to, saturated aliphatic carboxylic acid dihydrazides having 2 to 18 carbon atoms, carbohydrazide, thiocarbohydrazide, adipic dihydrazide, succinic dihydrazide, oxalyldihydrazide, ethylmalonic acid dihydrazide, malonic acid dihydrazide, glutaric acid dihydrazide, sebacic acid dihydrazide, maleic acid dihydrazide, fumaric acid dihydrazide, itaconic acid dihydrazide, terephthalic acid dihydrazide, isophthalic acid dihydrazide, pimelic acid dihydrazide, puromellitic acid dihydrazide. Trihydrazide conjugate compounds can include, but are not limited to, 1,2,4-butanetricarboxylic acid, trihydrazide, citric acid trihydrazide, 1,3,5-benzene(tricarboxylic trihydrazide), nitrilo-acetic trihydrazide, 1,2,4-benzene trihydrazide and cyanuric trihydrazide. Tetrathydrazide conjugate compounds can include, but are not limited to, ethylenediamine tetraacetic acid tetrahydrazide and 1,4,5,8-naphthoic acid tetrahydrazide.

A conjugate compound can comprise a polymer, referred to herein as a "conjugate polymer", which includes homopolymers and copolymers. In an aspect, a conjugate polymer comprises a non-absorbable polymer. Non-absorbable polymers can include polymers comprising one or more amine groups, one or more carboxylic acid groups, one or more thiol groups, one or more hydrazide groups or a combination thereof. Conjugate polymers disclosed herein can have two or more repeat units. In an aspect, disclosed polymers can have a molecular weight greater than 200 daltons. In an aspect, the molecular weight of a conjugate polymer is greater than about 1000. In an aspect, the molecular weight of a conjugate polymer is greater than about 5000. Conjugate polymers include, but are not limited to, polyalkylene oxide polymers that comprise one or more amine, carboxylic acid, thiol or hydrazide groups. Polyalkylene oxide conjugate polymers include, but are not limited to, methoxypolyethylene glycol, polyethylene glycol, polypropylene glycol or a copolymer of ethylene oxide and propylene oxide. In an aspect, a conjugate polymer can comprise one or more amine groups that can react with a carboxylic acid group of the UV absorbing compound. Conjugate polymers that comprise one or more amine groups include, but are not limited to,poly(ethyleneimine), poly(vinylamine) hydrochloride and copolymers thereof, poly(allylamine) and copolymers thereof, poly(4-aminostyrene) and copolymers thereof, poly(N-methylvinylamine) and copolymers thereof, poly(ethylene glycol) bis (2-aminoethyl), poly(2-Aminoethylmethacrylamide) and copolymers thereof, poly(N-(3-aminopropyl)methacrylamide) and copolymers thereof, 3arm PEG amine (glycerol core), 4arm PEG amine (pentaerythritol core), 6arm PEG amine (dipentaerythritol core), 8arm PEG amine (hexaglycerol core), 8arm PEG amine (tripentaerythritol core), methoxy PEG acetic acid, methoxy PEG butanoic acid, methoxy PEG hexanoic acid, methoxy PEG propionic acid, PEG (acetic acid)2, 4arm PEG acetic acid, 6arm PEG acetic acid, and 8arm PEG acetic acid.

In an aspect, a conjugate polymer can comprise one or more hydrazide groups that can react with a carboxylic acid group of the UV absorbing compound to form a UV absorbing conjugate compound with a -CO-NH-NH-CO- linkage. Conjugate polymers comprising one or more hydrazide groups include, but are not limited to, methoxy PEG hydrazide, PEG dihydrazide, 3arm PEG hydrazide, 4arm PEG hydrazide, 6 arm PEG hydrazide, 8arm PEG hydrazide, and poly(acryloyl hydrazide). Conjugate polymers comprising one or more hydrazide groups can be prepared from polymers that comprise a carboxylic acid group, acrylonitrile groups or an acrylamide group. Examples of these polymers are described in US 20070225453. Polyhydrazides can be obtained by reacting a polymer having a carboxylic acid lower alkyl ester group with hydrazine or hydrazine hydrate (see JPS52-22878B). Polyhydrazides can be obtained by reacting a polycarboxylic acid containing polymer with an excess of a dihydrazide compound such that the resulting polymer has residual hydrazide groups. These residual hydrazide groups can be reacted with a carboxylic acid moiety of a UV absorbing compound.

In an aspect, a conjugate polymer can comprise one or more thiol groups that can react with an acrylate, methacrylate, acrylamide or alkene group of a UV absorbing compound to form a UV absorbing conjugate compound with a thioether linkage. Polymers comprising one or more thiol groups include but are not limited to methoxy PEG thiol, PEG dithiol, 3arm PEG thiol, 4arm PEG thiol and 8arm PEG thiol,

In an aspect, a conjugate polymer can comprise one or more carboxylic acid groups that can react with an amine or hydrazide group of a UV absorbing compound to form a UV absorbing conjugate compound with an amide or -CO-NHNH-CO-linkage respectively. In an aspect, a non-degradable polymer comprising one or more carboxylic acid groups includes, but is not limited to, poly(acrylic acid) and copolymers thereof, poly(malic acid) and copolymers thereof, poly(maleic acid) and copolymers thereof, poly(fumaric acid) and copolymers thereof, poly(2-carboxyethyl acrylate) and copolymers thereof, poly(4-vinylbenzoic acid) and copolymers thereof, poly(maleic acid, mono-2-acryloxyethyl ester) and copolymers thereof, poly (methyacrylic acid) and copolymers thereof, and poly(phthalic acid, mono-2-acryloxyethyl ester) and copolymers thereof.

An UV absorbing conjugate polymer compound can be prepared by a free radical polymerization reaction of a monomer composition comprising at least one UV absorbing compound that is capable of undergoing free radical polymerization to produce UV absorbing conjugate compounds according to Formula 2 and/or 4 above. UV absorbing compounds that can under go free radical polymerization include, but are not limited to, UV absorbing compounds comprising a vinyl group, an acrylate group, a methacrylate group or an acrylamide group. UV absorbing compounds include, but are not limited to, N-[[3-(benzotriazol-2-yl)-2-hydroxy-5-(2,4,4-trimethylpentan-2-yl)phenyl]methyl]-2-methylprop-2-enamide (CAS 107479-06-1), and 2-[3-(2H-Benzotriazol-2-yl)-4-hydroxyphenyl]ethyl methacrylate (CAS 96478-09-0). In an aspect, monomers that are capable of undergoing free radical polymerization include, but are not limited to, monomers that comprise a vinyl group, an acrylate group, a methacrylate group or an acrylamide group. Monomers can include hydrophilic or hydrophobic monomers. Monomers that can be used include, but are not limited to, those supplied by Polysciences, Inc. The monomer to initiator ratio can be adjusted to change the molecular weight of the produced polymer.

A monomer composition disclosed herein can further comprise a crosslinking agent such that a crosslinked solid composition is produced. The crosslinked composition can undergo further processing such as milling, micronization, sieving or a combination thereof to produce microparticles or nanoparticles. In an aspect, an emulsion polymerization can be used to prepare crosslinked particles, and such polymerization reactions are known to those of skill in the art.

In an aspect, a conjugate polymer compound is degradable. In an aspect, a conjugate polymer compound is hydrolytically stable at pH 6.5 to pH 7.5 for a period of at least 6 months. In an aspect, a conjugate polymer compound can be cleaved enzymatically. In an aspect, a conjugate polymer compound is hydrolytically stable at pH 6.5 to pH 7.5 for a period of at least 6 months but is cleaved when exposed to an appropriate enzyme.

In an aspect, an enzymatically degradable conjugate polymer compound comprises a protein, a peptide or a polypeptide. In an aspect, the protein, peptide or polypeptide has one or more available amino groups to react with at least one UV absorbing compound. Such compounds include, but are not limited to, a protein, a peptide or a polypeptide that have one or more lysine moieties. Compounds that include one or more lysine moieties include, but are not limited to, poly(lysine).

In an aspect, a protein, peptide or polypeptide has one or more available thiol groups to react with the UV absorbing compound. Such conjugate polymer compounds include, but are not limited to, a protein, a peptide or a polypeptide that have one or more cysteine moieties. Conjugate polymer compounds that include one or more cysteines include but are not limited to poly(cysteine).

In an aspect, a protein, peptide or polypeptide has one or more available carboxylic acid groups to react with the UV absorbing compound. Such conjugate polymer compounds include, but are not limited to, a protein, a peptide or a polypeptide that have one or more carboxylic acid moieties. Conjugate polymer compounds that include one or more carboxylic acid include but are not limited to poly(aspartic acid) and copolymers thereof, poly(glutamic acid) and copolymers thereof.

In an aspect, a protein, peptide or polypeptide conjugate polymer compound has one or more available amine group and thiol group to react with the UV absorbing compound.

In an aspect, a conjugate polymer compound may comprise a polysaccharide. Polysaccharides that are enzymatically degradable and that have one or more functional groups that can react with a functional group on the UV absorbing compound or a derivative of a UV absorbing compound can be used to prepare the UV absorbing conjugate compound. Classes of polysaccharides that can be used in disclosed compositions include, but are not limited to, hyaluronic acid, alginic acid, cellulose, dextran, chitin, chitosan, xantham gum, xylan, guar gum, pullan, locust bean gum, starch, glucogen, cyclodextrin, amalose, amylopectin, pectin, callose, laminarin, chrysolaminarin, arabinoxylan, mannan, fucoidan and galalctomannan. These classes include derivatives and salts thereof.

For example, derivatives of cellulose, include but are not limited to cellulose esters, cellulose ethers and cellulose nitrates. Cellulose esters include but are not limited to cellulose acetate, cellulose acetate propionate (CAP), and cellulose acetate butyrate (CAB). Cellulose ethers include but are not limited to methyl cellulose, ethyl cellulose, hydroxyethyl cellulose, hydroxypropylcellulose, carboxymethyl cellulose, hydroxypropyl methyl cellulose, hydroxyethyl methyl cellulose, methyl ethyl hydroxyethyl cellulose, and ethyl hydroxyethyl cellulose. Salts of alginic acid include but are not limited to sodium alginate and calcium alginate.

In order for a polysaccharide or derivative thereof to react with an UV absorbing compound, the polysaccharide comprises at least one functional group that is capable of reaction with a functional group of the UV absorbing compound. Functional groups that can be used to react with UV absorbing compounds, include, but are not limited to, hydroxyl, carboxylic acid, thiol, amine, acrylate, methacrylate, vinyl, acrylamide, hydrazide, allyl and vinyl sulfone groups.

In an aspect, a polysaccharide comprises an amine group. The amine group of the polysaccharide can be reacted with a carboxylic acid group of an UV absorbing compound to form an amine group. The amine group of the polysaccharide can be reacted with an acrylate, acrylamide or methacrylate group of an UV absorbing compound through a Michael addition reaction. In an aspect, amine-containing polysaccharides include, but are not limited to, amino dextran, amino cyclodextrin, chitosan, amino cellulose, and deacetylated hyaluronic acid.

Amino dextran used can have a molecular weight of greater than 1000 daltons. Amino cyclodextrin compounds include but are not limited to 6-monodeoxy-6-monoamino-β-cyclodextrin hydrochloride (CAS 29390-67-8), heptakis-(6-amino-6-deoxy)-beta-cyclodextrin heptahydrochloride (CAS 65024-90-0), heptakis-(2,3-di-O-methyl-6-amino-6-deoxy)-β-cyclodextrin heptahydrochloride, 6-monoamino-6-monodeoxy-per-methyl-β-Cyclodextrin hydrochloride, A,D-6-diamino-6-dideoxy-β-cyclodextrin dihydrochloride, hexakis-(2,3-di-O-methyl-6-amino-6-deoxy)-α-cyclodextrin hexahydrochloride, A,D-6-diamino-6-dideoxy-α-cyclodextrin dihydrochloride, hexakis-(6-amino-6-deoxy)-α-cyclodextrin hexahydrochloride, octakis-(6-amino-6-deoxy)-γ-cyclodextrin octahydrochloride, octakis-(2,3-di-O-methyl-6-amino-6-deoxy)-γ-cyclodextrin octahydrochloride, 6- monoamino-6-monodeoxy-γ-cyclodextrin hydrochloride, and 6alpha-[(2-Aminoethyl)amino]-6a-deoxy-betacyclodextrin.

Amino cellulose includes, but is not limited to, 6-deoxy-6-(ω-aminoalkyl)aminocellulosecarbamates. Amino containing hyaluronic acid derivatives include, but are not limited to, deacetylated hyaluronic acid.

In an aspect, a polysaccharide comprises a thiol group. The thiol group of a polysaccharide can be reacted with an acrylate, acrylamide or methacrylate group of an UV absorbing compound through a Michael addition reaction to form a thioether. Thiolated cyclodextrins include, but are not limited to, heptakis-(6-deoxy-6-mercapto)-beta-cyclodextrin (CAS 160661-60-9), hexakis-(6-deoxy-6-mercapto)-α-cyclodextrin, and octakis-(6-deoxy-6-mercapto)-γ-cyclodextrin.

Thiolated chitosan can include, but is not limited to, alkyl thiolated chitosan derivatives such as chitosan-cysteine, chitosan-thiobutylamidine, chitosan-thioglycolic acid, chitosan-N-acetylcysteine, and chitosan-thioethylamidine and the aryl thiolated chitosan such as chitosan-6-mercaptonicotinic acid and chitosan-4-mercaptobenzoic acid. The preparation of thiolated chitosan derivatives are detailed in WO2015169728A1, Kast, Constantia E; Frick, Wolfram; Losert, Udo; Bernkop-Schnürch, Andreas, International Journal of Pharmaceutics, , Volume 256 (1) - Apr 30, 2003, Roldo, Marta & Hornof, Margit & Caliceti, Paolo & Bernkop-Schnürch, Andreas. (2004). Thiolated hyaluronic acid can be prepared according to US 20100330143, US 20080031854. US 20100144902, and US 20100152423.

In an aspect, the polysaccharide can comprise one or more hydrazide groups. In an aspect, the hydrazide comprising polysaccharide is derived from a polysaccharide that comprises one or more carboxylic acid groups wherein one or more of the carboxylic acid groups have been reacted with a dihydrazide compound such that the derivatized polysaccharide comprises one or more hydrazide groups. In an aspect, polysaccharides that comprise one or more carboxylic acid groups include but are not limited to hyaluronic acid, galacturonan, xylogalacturonan, apiogalacturonan, alginic acid, carboxymethyl cellulose, and cellulose acetate phthalate, In an aspect, the hydrazide polysaccharide can include but is not limited to hydrazide derivatized hyaluronic acid, hydrazide derivatized carboxymethyl cellulose, hydrazide derivatized cellulose acetate phthalate, hydrazide derivatized, alginic acid and hydrazide derivatized galacturonan.

Hydrazide functionalized hyaluronic acid can be prepared according to US 20100330143, US 20100144902, and US 20100152423.

In an aspect, the polysaccharide can be degraded enzymatically. Hyaluronic acid can be degraded by a hyaluronidase, chitosan can be degraded by lysozyme, cellulose based polymers and oligomers can be degraded by cellulases and endoglucanases, and dextrans can be degraded by dextranase.

In an aspect, a conjugate polymer compound may comprise polyamino acids. Polyamino acids can comprise peptides and/or proteins. Peptides include compounds with two or more amino acids. UV absorbing compounds with a carboxylic acid group or a sulfonic acid group can be reacted with the terminal amine group of a peptide or protein to form an amide or sulfonamide linkage respectively between the UV absorbing compound and the peptide or protein.

UV absorbing conjugate compounds with two or more UV absorbing compounds attached can be prepared using proteins or peptides that comprise one or more amine containing amino acids. Suitable amine-containing amino acids include lysine, arginine and histidine. UV absorbing compounds with a carboxylic acid group or a sulfonic acid group can be reacted with the terminal amine group and the amine group of the amine containing amino acid of the peptide or protein to form an amide or sulfonamide linkage respectively between the UV absorbing compound and the peptide or protein.

UV absorbing compounds with a hydrazide group can be reacted with the terminal carboxylic acid group of a peptide or protein to form a linkage between the UV absorbing compound and the peptide or protein. UV absorbing compounds with a hydrazide group can be reacted with the terminal carboxylic acid group of a peptide or protein as well as with any carboxylic acid containing amino acid within the peptide or protein to form a linkage between the UV absorbing compound and the peptide or protein. Suitable carboxylic acid containing amino acid include one or more aspartic acid and glutamic acid. In an aspect, the peptide is poly(glutamic acid), poly(aspartic acid) or combinations thereof. In aspect, the conjugate compound can be a hydrolyzed collagen. In an aspect, the hydrolyzed collagen can be gelatin that comprise glutamic acid.

UV absorbing conjugate compounds with two or more UV absorbing compounds attached can be prepared using proteins or peptides that comprise one or more amine-containing amino acids. Suitable amine containing amino acids include lysine, arginine and histidine. In an aspect, the peptide is poly(lysine), polyarginine, or poly(histidine) or combinations thereof. In aspect, the conjugate compound can be a hydrolyzed collagen. In an aspect, the hydrolyzed collagen can be gelatin that comprises arginine.

UV absorbing compounds with an alkene group can react with peptides or proteins that contain one or more free thiol groups. In an aspect, the peptide or protein comprise one or more cysteine groups in which the thiol is in a free form and not part of a disulfide bond.

UV absorbing conjugate compounds comprising polysaccharides or polyamino acids can be crosslinked. Suitable crosslinkers include but are not limited to compounds that comprise two or more vinyl sulfone groups, epoxide groups, isocyanate groups or carbodiimide groups. The crosslinked material can undergo further processing such as milling, micronization, sieving or a combination thereof to produce microparticles or nanoparticle. In an aspect, the crosslinking reaction can take place as an emulsion such that crosslinked particles are produced.

In another aspect, polysaccharide conjugate compounds or polyamino acid conjugate compounds can be formed into crosslinked particle via an emulsion reaction or through a crosslinking reaction with subsequent processing such as milling, micronization, sieving or a combination thereof.

Polysaccharides or polyamino acids can be crosslinked using a suitable crosslinker. In an aspect, the crosslinking reaction can be an emulsion reaction such that particles are formed. In an aspect, the crosslinked material can be converted into a particulate form by processes that include milling, micronization, sieving or a combination thereof. The crosslinked particles can be reacted with a UV absorbing compound as described herein such that the UV absorbing compound is chemically bound to pre-crosslinked particles.

A UV absorbing conjugate compound can be incorporated into a polysaccharide or polyamino acid prior to the crosslinking reaction and then it can be entrapped within the crosslinked material. In an aspect, the crosslinking reaction can be an emulsion reaction such that particles are formed. In an aspect, the crosslinked material can be converted into a particulate form by processes that include milling, micronization, sieving or a combination thereof.

The UV absorbing compound comprising a hydrazide group can react with the residual carboxylic acid group of a degradable polyester using a carbodiimide to activate the ester. In an aspect, an initiator that comprises both at least one hydroxyl group and at least one carboxylic acid group can be used to initiate a ring opening polymerization of a polyester. Monomers that can be used include glycolide, lactide, ε-caprolactone, trimethylene carbonate, p-dioxanone, 1,5-dioxepan-2-one and morpholinedione. Initiators that can be used include but are not limited to hydroxy alkane carboxylic acid compounds. Examples of hydroxy alkane carboxylic acid compounds include, but are not limited to, 10-hydroxydecanoic acid, 12-hydroxydodecanoic acid, 11-hydroxyundecanoic acid, 15-hydroxypentadecanoic acid, 16-hydroxyhexadecanoic acid, 12-hydroxyoctadecanoic acid, 12-hydroxystearic acid, citric acid, glycolic acid, and tartaric acid. The molecular weight of the polyester greater than 1000 daltons. In an aspect, the molecular weight is greater than 1500.

### UV absorbing compounds

UV absorbing compounds absorb UV radiation with a wavelength of less than 400 nm, e.g., from 200 to 400 nm. UV absorbing compounds can absorb, for example, UV-A (from 320 to 400 nm), UV-B (from 290 to 319 nm) and/or UV-C (from 200 to 289 nm) light. In an aspect, UV absorbing compounds absorb UV-A and/or UV-B radiation. In an aspect, UV absorbing compounds that absorb UV-A and/or UV-B radiation and deactivate the absorbed radiation energy in a nonradiative manner. UV absorbing compounds include, but are not limited to, benzophenone-based compounds, benzotriazole-based compounds, benzimidazole-based compounds that have absorbance in the 200 to 400 nm range. In an aspect, the UV absorbing compounds include, but are not limited to, benzophenone-based compounds, benzotriazole-based compounds, benzimidazole-based compounds that have absorbance in the 200 to 380 nm range. In an aspect, the UV absorbing compounds include, but are not limited to, benzophenone-based compounds, benzotriazole-based compounds, benzimidazole-based compounds that have absorbance in the 200 to 350 nm range.

Disclosed herein are UV absorbing compounds comprising acrylamide, acrylate, methacrylate, maleimide, acrylonitrile, vinyl sulfone, amine, sulfonic acid, allyl, hydrazide and/or carboxylic acid groups that are available for reaction with a conjugate compound. In an aspect, UV absorbing compounds that comprise an acrylamide group, acrylate group, methacrylate group, maleimide group, acrylonitrile groups or a vinyl sulfone group that can undergo a Michael addition reaction can be used to prepare conjugate compounds. UV absorbing compounds that contain one or more functional groups capable of a Michael addition reaction can be conjugated to compounds that comprise one or more amine groups, thiol groups or a combination thereof. UV absorbing compounds that can be used include, but are not limited to, UV absorbing benzotriazole compound that comprise an acrylamide group, acrylate group, methacrylate group, maleimide group, acrylonitrile groups or a vinyl sulfone group. Examples of these compounds include, but are not limited to, N-[[3-(benzotriazol-2-yl)-2-hydroxy-5-(2,4,4-trimethylpentan-2-yl)phenyl]methyl]-2-methylprop-2-enamide (CAS 107479-06-1), and 2-[3-(2H-Benzotriazol-2-yl)-4-hydroxyphenyl]ethyl methacrylate (CAS 96478-09-0).

In an aspect, disclosed herein are UV absorbing compounds that comprise a carboxylic acid group that can undergo a reaction with an amine group of a conjugating compound to form an amide bond. UV absorbing compounds include, but are not limited to, benzotriazole compounds that comprise a carboxylic acid group, including, but not limited to, 3-[3-(2H-Benzotriazol-2-yl)-5-tert-butyl-4-hydroxyphenyl]propionic acid (CAS 84268-36-0).

An UV absorbing compound can comprise a hydrazide group. A hydrazide comprising UV absorbing compound can be reacted with a conjugate compound that comprises one or more carboxylic acid groups. In an aspect, a hydrazide UV absorbing compound is prepared by reacting 3-[3-(2H-Benzotriazol-2-yl)-5-tert-butyl-4-hydroxyphenyl]propionic acid with an excess of a dihydrazide. The reaction product will comprise a hydrazide group that is chemically bound to the UV absorbing compound.

In an aspect, UV absorbing compounds that comprise an alkene group that can undergo a thiol-ene reaction with a thiol group of a conjugate compound. An alkene containing UV absorbing compound includes, but is not limited to, benzotriazole compounds that comprise an alkene group. Benzotriazole compounds that comprise an alkene group include, but are not limited to, N-[[3-(benzotriazol-2-yl)-2-hydroxy-5-(2,4,4-trimethylpentan-2-yl)phenyl]methyl]-2-methylprop-2-enamide (CAS 107479-06-1), 2-[3-(2H-Benzotriazol-2-yl)-4-hydroxyphenyl]ethyl methacrylate (CAS 96478-09-0) and 2-(2H-Benzotriazol-2-yl)-4-methyl-6-(2-propenyl)phenol (CAS 2170-39-0). In an aspect, the thiol-ene reaction can occur in the presence of a photo-initiator and a light source. In an aspect, the light source emits ultra-violet radiation.

Thiol-ene reactions can be initiated by cleavage type photoinitiators, also called Norrish Type I photoinitiators, and H-abstraction initiators, also called Norrish Type II photoinitiators. Cleavage type photoinitiators include, but are not limited to, 2,2-dimethoxy-1,2-diphenylethan-1-one, 2-Hydroxy-2-methyl-1-phenylpropanone, 1-hydroxy-cyclohexylphenylketone, 2, 4, 6-trimethylbenzoyl diphenylphosphine oxide (TMDPO) and 2, 2-dimethoxy-2-phenyl acetophenone (DMPA). H-abstraction type photoinitiators include, but are not limited to, benzophenone (B), thioxanthone (TX), isopropyl thioxanthone and camphorquinone (CQ). Photoinitiators also include the Igacure series of photoinitiators from BASF/Ciba.

The thiol-ene reactions can be initiated by a thermal initiator. Thermal initiators include, but are not limited to, 2,2'-azobis(isobutyronitrile) (AIBN) and benzoyl peroxide.

In an aspect, UV absorbing compounds comprising a sulfonic acid group can undergo a reaction with an amine group of a conjugating compound to form a sulfonamide bond. Sulfonic acid containing UV absorbing compounds include, but are not limited to, benzimidazole compounds that comprise a sulfonic acid group, benzotriazole compounds that comprise a sulfonic acid group, and benzophenone compounds that comprise a sulfonic acid group. Examples of benzimidazole compounds that comprise a sulfonic acid group include, but are not limited to, 2-phenylbenzimidazole-5-sulfonic acid or Ensulizole (CAS 27503-81-7). Examples of benzophenone compounds that comprise a sulfonic acid group include, but are not limited to, sulisobenzone or benzophenone-4 (CAS 4065-45-6). Examples of benzotriazole compounds that comprise a sulfonic acid group include, but are not limited to, sodium;3-(benzotriazol-2-yl)-5-butan-2-yl-4-hydroxybenzenesulfonate (CAS 92484-48-5), 3-(benzotriazol-2-yl)-5-butan-2-yl-4-hydroxybenzenesulfonic acid, 2-(2-hydroxy-3,5-dimethylphenyl)benzotriazole-4-sulfonic acid, 2-(5-tert-butyl-2-hydroxyphenyl)benzotriazole-5-sulfonic acid, 2-(5-Tert-butyl-2-hydroxy-3-propan-2-ylphenyl)benzotriazole-5-sulfonic acid, 2-(2-hydroxy-3-tert-butyl-5-methylphenyl)-2H-benzotriazole-5-sulfonic acid, and 3-(benzotriazol-2-yl)-5-tert-butyl-4-hydroxybenzenesulfonic acid.

Disclosed UV absorbing conjugate compounds may be formed into or incorporated into a particle, a sphere, a hollow sphere, a fiber, a hollow fiber or a liposome. Disclosed UV absorbing conjugate compounds may be incorporated into a particle, a sphere or a hollow sphere, a fiber or a hollow fiber formed from a matrix material. In an aspect, the matrix material may be a polymer, a glass or an inorganic matrix. The disclosed UV absorbing conjugate compounds may be coated onto a particle, a sphere or a hollow sphere, a fiber or a hollow fiber formed from a matrix material. Disclosed UV absorbing conjugate compounds may added into the hollow space of a hollow sphere, a hollow fiber or a porous inorganic matrix. In an aspect, a particle, a sphere, a hollow sphere, or a liposome has a mean diameter of less than about 500 nm. In an aspect, a particle, a sphere, a hollow sphere, or a liposome has a mean diameter of less than about 400 nm. In an aspect, a particle, a sphere, a hollow sphere, or a liposome has a mean diameter of less than about 300 nm.

UV absorbing conjugate compounds disclosed herein can be incorporated into formulation compositions that can be applied to one or more body surfaces, such as a sunscreen, a cosmetic product or a hair product. Sunscreen compositions can include, but are not limited to, daily use sunscreens, water resistant sunscreens, or combinations thereof. Cosmetic compositions can include, but are not limited to, formulations such as a moisturizer, foundation, lip stick, lip gloss, chap stick, concealer, highlighter, blushes, eye shadows, cleansers, toners, serums, anti-aging products, setting sprays or combinations thereof. Hair product compositions include, but are not limited to, shampoos, conditioners, leave-in conditioners, hair mousse, hair gels, hair spray, curling creams, hair waxes, treatment oils, medicated hair treatments or combinations thereof.

Disclosed formulation compositions comprising UV absorbing conjugate compounds may be prepared by methods that are well known by a person of ordinary skill in the field of cosmetic formulation. Various forms of formulation compositions are known. These forms include, but are not limited to, solutions, suspensions, emulsions, liposomes, dispersions, particulates. Product forms for the sunscreen, cosmetic or hair formulation can include but is not limited to solutions, sprays, gels, lotions, creams, mousses, emulsions, sticks, powders, or combinations thereof.

In an aspect, a formulation composition disclosed herein, e.g., a sunscreen, cosmetic or hair formulation, can be an emulsion. In an aspect, a formulation is an oil-in-water (o/w) emulsion with a continuous water phase and a discontinuous oil phase. A moisturizer or sunscreen lotion is an example of an oil-in-water composition. In an aspect, a formulation is a water-in-oil (w/o) emulsions with a continuous oil phase and a discontinuous water phase. A sunscreen cream formulation composition may be a water-in-oil composition. In an aspect, an UV absorbing conjugate compound can be in the water phase. In an aspect, an UV absorbing conjugate compound can be in the oil phase. In an aspect, an UV absorbing conjugate compound can be in the water phase and the oil phase. In an aspect, a formulation composition can comprise two or more different UV absorbing conjugate compounds with one UV absorbing conjugate compounds in the water phase and the other in the oil phase. In an aspect, a formulation comprises two or more different UV absorbing conjugate compounds, optionally, with one UV absorbing conjugate compound in the water phase and the other in the oil phase and/or the water phase. In an aspect, a formulation comprises two or more different UV absorbing conjugate compounds with one UV absorbing conjugate compounds in the oil phase and the other in the oil phase and/or the water phase.

For example, oil-in-water emulsions may comprise a water content of about 40% (w/w) to 80% (w/w) of the final formulation. In an aspect, the water content can be about 50% (w/w) to about 70% (w/w) of the final formulation.

### Emusifiers

Formulation compositions disclosed herein comprise one or more UV absorbing conjugate compounds may comprise an emulsifier. Water-in-oil emulsifiers include, but are not limited to, glyceryl stearate, lecithin, polyglyceryl oleate, sorbitan stearate, glycol stearate, glyceryl oleate, sorbitan oleate, laureth-3, PEG-8 beeswax, glycol distearate, shea butter glycerides, methyl glucose dioleate, hydroxylated lanolin, and emulsifiers sold by Evonik. See at personal-care.evonik.com/product/personal-care/en/products-solutions/products/pages/default.aspx?category=3591.

Oil-in-water emulsifiers include, but are not limited to, ceteareth-20, ceteareth-25, gum arabic, PEG-7 glyceryl cocoate, PEG-40 hydrogenated castor oil, polysorbate 20, polysorbate 60, polysorbate 80, PEG-150 distearate, cetearyl alcohol, stearic acid, glyceryl stearate citrate, laneth-16, ceteth-16, oleth-16, steareth-16, stearyl alcohol, emulsifiers sold by Evonik. See at personal-care.evonik.com/product/personal-care/en/products-solutions/products/pages/default.aspx?category=3496.

A formulation composition comprising one or more UV absorbing conjugate compounds may comprise an emollient. Emollients can include but are not limited to petrolatum, silicone oils, castor oil, lanolin, cocoa butter, liquid paraffin, cetyl alcohol, cetearyl alcohol, isopropyl myristate, isopropyl palmitate, shea butter, stearic acid, steryl alcohol, vegetable oil and combinations thereof.

A formulation composition comprising one or more UV absorbing conjugate compounds may comprise a humectant. A humectant can include, but is not limited to, algae extract, aloe vera, aloe vera palmitate, butylene glycol, caprylyl glycol, ethoxydiglycol, glycerin, hexanediol, honey, hyaluronic acid, methyl gluceth-10, pentylene glycol, propanediol, prolylene gycol, sorbitol, sucrose cocoate, urea, sodium lactate and combinations thereof.

A formulation composition comprising one or more UV absorbing conjugate compounds may comprise a conditioning agent. Conditioning agents include but are not limited to cocamidopropyl betaine, cocamidopropyl betaine, stearamidopropyl dimethylamine, trioctyldodecyl citrate, trioctyldodecyl citrate, PEG/PPG-8/3 diisostearate, myristamidopropyl dimethylamine phosphate (and) propylene glycol, polyquaternium-6, polyquaternium-47, polyquaternium-53, polyquaternium-7, polyquaternium-10, polyquaternium-22, polyquaternium-39, polyquaternium-5, acetylated lanolin, cetearyl alcohol (and) cetrimonium bromide, soyamidopropalkonium chloride, cocamidopropyl dimethylamine, isostearamidopropyl dimethylamine, acetamide MEA, isostearamidopropyl laurylacetodimonium chloride (and) propylene glycol, isostearamidopropyl ethyldimonium ethosulfate (and) propylene glycol, starch hydroxypropyl trimonium chloride, PEG-7 amodimethicone, PEG-33 (and) PEG-8 dimethicone (and) PEG-14, dimethiconol stearate, dimethicone PEG-8 phosphate, silicone quaternium-8, dimethicone PEG-7 cocoate, dimethicone PEG-8 beeswax, and combinations thereof.

A formulation composition comprising one or more UV absorbing conjugate compounds may comprise inorganic particulates. Inorganic particles can include but are not limited to glass particles, glass beads, dyes, zinc oxide and titanium dioxide.

A formulation composition comprising one or more UV absorbing conjugate compounds may comprise compounds that enhance water resistance of the formulation composition. Compounds that enhance water resistance of a formulation include, but are not limited to, film-forming polymers. Polymers that can be used include but are not limited to dehydroxanthan gum, Dermacryl AQF polymers, Bis-PEG-18methyl ether dimethylsilane, trimethylsiloxysilicate, and butylated PVP (polyvinylpyrrolidone), octylacrylamide/acrylates copolymer, octylacrylamide/acrylate/butylaminoethyl methacrylate copolymer, film forming polymers suitable for use in the present invention include: from National Starch and Chemical Company, AMPHOMER and AMPHOMER LV-71 polymers (octylacrylamide/acrylates/butylaminoethyl methacrylate copolymer), AMPHOMER HC polymer (acrylates/octylacrylamide copolymer) BALANCE 0/55 and BALANCE CR polymers (acrylates copolymer), BALANCE 47 polymer (octylacrylamide/acrylates/butylaminoethyl methacrylate copolymer), RESYN 28-2930 polymer (VA/crotonate/vinyl neodecanoate copolymer), RESYN 28-1310 polymer (VA/Crotonate copolymer), DynamX polymer (polyurethane-14 (and) AMP-Acrylates copolymer), RESYN XP polymer (acrylates/octylacrylamide copolymer), STRUCTURE 2001 (acrylates/steareth-20 itaconate copolymer) STRUCTURE 3001 (acrylates/ceteth-20 itaconate copolymer), YODOSOL 32A707, YODOSOL GH15, YODOSOL GH32, YODOSOL GH33, YODOSOL GH34, YODOSOL GH35, YODOSOL GH256, YODOSOL GH800, YODOSOL GH810, YODOSOLGH32A707F, YODOSOL GH15F, YODOSOL GH34F, YODOSOL GH800F, YODOSOL GH810F, YODOSOL GH800PF (acrylates copolymer), YODOSOL GH52, YODOSOL GH52-OP (styrene/methacrylamide/acrylates copolymer), YODOSOL GH265 (polyacrylate-2), YODOSOL GH840, YODOSOL GH41F, YODOSOL GH4I (styrene/acrylates copolymer), YODOSOL PUD (polyurethane-10 (and) PEG-12 dimethicone (and) alcohol), DERMACYL AQF (acrylates copolymer), DERMACRYL C (proposed: acrylates copolymer) and DERMACRYL 79 and LT polymers (acylates/octyacrylamide copolymer); from ISP, OMNIREZ-2000 (PVM/MA half ethyl ester copolymer), GANTREZ A-425 (butyl ester of PVM/MA copolymer), GANTREZ AN-119 PVM/MA copolymer, GANTREZ ES 225 (ethyl ester of PVM/MA copolymer), GANTREZ ES-425 (butyl ester of PVM/MA copolymer), AQUAFLEX XL-30 (Polyimide-1), ALLIANZ LT-120 (Acrylates/C1-2 Succinates/Hydroxyacrylates Copolymer), ALLIANZ OPT (Acrylates/C12-22 Alkyl Methacrylate Copolymer), STYLEZE CC-10 (PVP/DMAPA Acrylates Copolymer), STYLEZE 2000 (VP/Acrylates/Lauryl Methacrylate Copolymer), STYLEZE W-20 (Polyquaternium-55), ADVANTAGE PLUS (VA/Butyl Maleate/Isobornyl Acrylate Copolymer); from BASF, ULTRAHOLD STRONG (acrylic acid/ethyl acrylate/t-butyl acrylamide), LUVIMER 100 P (t-butyl acrylate/ethyl acrylate/methacrylic acid), LUVIMER 36D (ethyl acrylate/t-butyl acrylate/methacrylic acid), LUVISET PUR (Polyurethane-1), LUVISET Clear (VP/Methacrylamide/Vinyl Imidazole Copolymer), LUVIFLEX SOFT (Acrylates Copolymer), ULTRAHOLD 8 (Acrylates/Acrylamide Copolymer), LUVIFLEX Silk (PEG/PPG-25/25 Dimethicone/Acrylates Copolymer), LUVISET CAN (VA/crotonate/vinyl neodecanoate copolymer), LUVIMER PRO55 (acrylates copolymer); from Amerchol, AMERHOLD DR-25 (acrylic acid/methacrylic acid/acrylates/methacrylates); from Rohm and Haas, ACUDYNE 258 (acrylic acid/methacrylic acid/acrylates/methacrylates/hydroxy ester acrylates), ACUDYNE DHR (acrylates/hydroxyesters acrylates copolymer) ALLIANZ OPT (Acrylates/C12-22 Alkyl Methacrylate Copolymer); from Mitsubishi and distributed by Clariant, DIAFORMER Z-301, DIAFORMER Z-SM, and DIAFORMER Z-400 (methacryloyl ethyl betaine/acrylates copolymer), ACUDYNE 180 (Acrylates/Hydroxyesters Acrylates Copolymer), ACUDYNE SCP (Ethylenecarboxyamide/AMPSA/Methacrylates Copolymer), and the ACCULYN rheological modifiers; from ONDEO Nalco, FIXOMER 40 (acrylates copolymer), FIXOMER A-30 and FIXOMER N-28 (INCI names: methacrylic acid/sodium acrylamidomethyl propane sulfonate copolymer); from Eastman Chemical, Eastman polymer AQ38S and AQ55S (diglycol/CHEM/isophthalates/SIP copolymer); from Interpolymer, vinylpyrrolidone/tricontanyl copolymers available as GANEX WP660 from ISP, SYNTRAN 5009 AND SYNTRAN 5760 (Styrene/Acrylates/Ammonium Methacrylate Copolymer), SYNTRAN 5190 (acrylates copolymer), SYNTRAN 5900 and 5902 (polystyrene), SYNTRAN 5903, 5904, 5905 (styrene/acrylates copolymer), SYNTRAN KL-219C (ammonium acrylates copolymer), SYNTRAN PC 5112 (polyacrylate-16), SYNTRAN PC5208 (polyacrylate-15), SYNTRAN PC5100 (Polyacrylate-21 and Acrylates/Dimethylaminoethyl Methacrylate Copolymer) SYNTRAN PC5107 and PC5117 (Polyacrylate-18 and Polyacrylate-19), SYNTRAN PC5205 and PC5227 (Polyacrylate-15 and Polyacrylate-17); from Noveon, FIXATE G-100 (AMP-Acrylates/Allyl Methacrylate Copolymer), FIXATE PLUS (Polyacrylates-X), CARBOPOL Ultrez 10 (Carbomer), CARBOPOL Ultrez 20 (Acrylates/C10-30 Alkyl Acrylates Copolymer), AVALURE AC series (Acrylates Copolymer), AVALURE UR series (Polyurethane-2, Polyurethane-4, PPG-17/IPDI/DMPA Copolymer); from Inolex Chemical Company, LEXOREZ TL8 (Trimethylpentanediol/Adipic Acid Copolymer, LEXOREZ TC8 and LEXOREZ TC-1 (INCI names: Trimethylpentanediol/Adipic Acid/Isononanoic Acid Copolymer), LEXOREZ 200 (Trimethylpentanediol/Adipic Acid/Glycerin Crosspolymer), LEXOREZ 100 (Adipic Acid/Diethylene Glycol/Glycerin Crosspolymer), copolymer of vinylpyrrolidone and a long-chain alpha.-olefin, such as those commercially available from ISP Specialty Chemicals of Wayne, N.J. as GANEX V220; LEXFILM SUN (polyester-7 (and) neopentyl glycol diheptanoate), LEXFILM SPRAY (polyester-10 (and) propylene glycol dibenzoate); from Dow Corning: DOW CORNING FA 4002 ID SILICONE ACRYLATE (Isododecane (and) Acrylates/Polytrimethylsiloxymethacrylate Crosspolymer), DOW CORNING FA4001 ID SILICONE ACRYLATE (Cyclopentasiloxane (and) Acrylates/Polytrimethylsiloxymethacrylate Copolymer); and any combination of the foregoing. hydrogenated dimer dilinoleyl/dimethylcarbonate copolymer, available from Cognis Corporation of Ambler, Pa. as COSMEDIA DC; Film forming polymers can include polymers with no or limited water solubility as described in WO2017048706. The amount of film-forming polymer present in the composition may be from about 0.1% to about 5%, or from about 0.1% to about 3%, or from about 0.1% to about 2%.

A formulation composition disclosed herein may comprise one or more UV absorbing conjugate. In an aspect, an UV absorbing conjugate compound is present in an amount effective to provide a SPF of about 10 or greater. In an aspect, the amount of one or more UV absorbing conjugate compounds in a formulation composition may vary from about 2% (w/w) to about 60% (w/w) of the final formulation. In an aspect, the amount of one or more UV absorbing conjugate compound present in a formulation can be from about 6% (w/w) to about 40% (w/w) of the final formulation. In an aspect, the amount of one or more UV absorbing conjugate compounds in a formulation can be from about 6% (w/w) to about 25% (w/w) of the final formulation.

Formulation compositions, such as sunscreen, cosmetic and hair compositions disclosed herein can comprise components, that include, but are not limited to, antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, skin healing agents, SPF boosting agents or combinations thereof. In an aspect, the SPF boosting agent may be an agent that can reflect or refract UV light. In an aspect, the SPF boosting agent may be a hollow particle, a hollow sphere, a hollow fiber, a porous particle, a porous sphere or a porous fiber. In an aspect, the SPF boosting agent may comprise a polymer. In an aspect, the polymer may be a degradable polymer or a non-degradable polymer. In an aspect, the polymer may be a styrene/acrylate co-polymer.

Formulation compositions, such as sunscreen, cosmetic and hair compositions disclosed herein can have a pH that is from about 4.0 to about 8.0, or such as from about 5.5 to about 7.0.

Compositions and formulation compositions can be used to ameliorate the effects of UV radiation on body surfaces, particularly human and animal skin surfaces. For example, an effective amount of a formulation composition disclosed herein comprising a UV absorbing conjugate compound composition is applied to the skin in an amount and frequency to provide at least SPF 15 protection for human or animal skin.

### Kits

The present disclosure comprises a kit comprising a formulation composition comprising an UV absorbing conjugate compound composition disclosed herein, contained within a container. The kit may further comprise written instructions for its use.

Disclosed herein are compositions, methods, and kits comprising one or more UV absorbing conjugate compound compositions disclosed herein, contained within a container. The kit may further comprise written instructions, which optionally may be located on or within the container, for its use.

Disclosed herein are topical formulation compositions comprising one or more UV absorbing conjugate compounds comprising a UV absorbing compound conjugated with a conjugate compound, wherein the a UV absorbing compound is selected from a group consisting of a benzophenone compound conjugated to a conjugate compound via a sulfonamide linker wherein the benzophenone conjugate compound has a molecular weight of at least 800 daltons; a benzimidazole compound conjugated to a conjugate compound via a sulfonamide linker wherein the benzimidazole conjugate compound has a molecular weight of at least 800 daltons; a benzotriazole compound conjugated to a conjugate compound via an amide linker wherein the benzotriazole conjugate compound has a molecular weight of at least 800 daltons; a benzotriazole compound conjugated to a [conjugate compound through the product of a Michael addition reaction wherein the benzotriazole conjugate compound has a molecular weight of at least 800 daltons; a benzotriazole functionalized polymer wherein benzotriazole functionalized polymer has a molecular weight of at least 800 daltons and wherein the benzotriazole is bonded to the polymer as a methacrylate, acrylate or acrylaminde derivative of a benzotriazole compound; and a benzotriazole compound conjugated to a polysaccharide wherein the benzotriazole polysaccharide conjugate compound has a molecular weight of at least 800 daltons, wherein the UV absorbing conjugate compound is not transported or absorbed through the skin. UV absorbing compounds may comprise 1) (U -X)n -C, wherein, U is an UV absorbing compound moiety, X is a thioether, amine, amide, urethane, sulfonamide group, or - CO-NH-NH-CO-, C is a conjugate compound moiety and n is an integer and where n is ≥1; or 2) (U -X)n -C, wherein, U is an UV absorbing compound moiety, X is an ester, C is a conjugate compound moiety and n is an integer and where n is ≥1; or 3) wherein the UV absorbing conjugate compound has the structure, 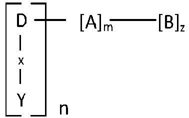 wherein Y is an UV absorbing compound moiety, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n is an integer and where n is ≥1 and m and z are each an integer and where m is ≥0, and z is ≥0, and the structure can be a block copolymer or it can be a random copolymer; or 4) wherein the UV absorbing conjugate compound comprises (U -X)n -C-(X-U1)m, wherein, U is an UV absorbing compound moiety, U1 is a UV absorbing compound moiety that has a different chemical structure to U, X is a thioether, amine, amide, ester, urethane, sulfonamide group, -CO-NH-NH-CO-, C is a conjugate compound moiety and n and m are each an integer and where n and m is ≥1; 5) wherein the UV absorbing conjugate compound comprises, 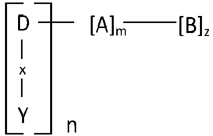 wherein Y is an UV absorbing compound moiety, Y1 is a UV absorbing compound moiety that has a different chemical structure to Y, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n and p are each an integer and where n and p is ≥1 and m and z are each an integer and where m is ≥0, and z is ≥0, and the structure can be a block copolymer or it can be a random copolymer.

In general, UV absorbing conjugate compounds comprise a UV absorbing compound comprising benzophenone-based compounds, benzotriazole-based compounds, or benzimidazole-based compounds that have absorbance from about 200 to about 380 nm range. UV absorbing conjugate compounds may comprise one or more UV absorbing benzotriazole compounds comprising the reaction residues of an acrylamide group, acrylate group, methacrylate group, maleimide group, acrylonitrile groups or a vinyl sulfone group.

Topical formulation compositions disclosed herein may comprise a sunscreen formulation comprising a daily use sunscreen, a water resistant sunscreen, or combinations thereof; a cosmetic formulation comprising a moisturizer, foundation, lip stick, lip gloss, chap stick, concealer, highlighter, a blush, eye shadows, cleansers, toners, serums, anti-aging products, setting sprays or combinations thereof; a hair product composition include, comprising, a shampoo, a conditioner, a leave-in conditioner, a hair mousse, a hair gel, a hair spray, a curling cream, a hair wax, a treatment oil, a medicated hair treatment or combinations thereof.

### Definitions

As used herein, nomenclature for compounds, including organic compounds, can be given using common names, IUPAC, IUBMB, or CAS recommendations for nomenclature. When one or more stereochemical features are present, Cahn-Ingold-Prelog rules for stereochemistry can be employed to designate stereochemical priority, *EIZ* specification, and the like. One of skill in the art can readily ascertain the structure of a compound if given a name, either by systemic reduction of the compound structure using naming conventions, or by commercially available software, such as CHEMDRAW^{™} (Cambridgesoft Corporation, U.S.A.).

As used in the specification and the appended claims, the singular forms "a," "an" and "the" include plural referents unless the context clearly dictates otherwise. Thus, for example, reference to "a functional group," "an alkyl," or "a residue" includes mixtures of two or more such functional groups, alkyls, or residues, and the like.

References in the specification and concluding claims to parts by weight of a particular element or component in a composition denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent (wt. %) of a component, unless specifically stated to the contrary, is based on the total weight of the formulation or composition in which the component is included.

As used herein, when a compound is referred to as a monomer or a compound, it is understood that this is not interpreted as one molecule or one compound. For example, two monomers generally refers to two different monomers, and not two molecules.

As used herein, the terms "optional" or "optionally" means that the subsequently described event or circumstance can or cannot occur, and that the description includes instances where said event or circumstance occurs and instances where it does not.

As used herein, the terms "about," "approximate," and "at or about" mean that the amount or value in question can be the exact value designated or a value that provides equivalent results or effects as recited in the claims or taught herein. That is, it is understood that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. In such cases, it is generally understood, as used herein, that "about" and "at or about" mean the nominal value indicated ±10% variation unless otherwise indicated or inferred. In general, an amount, size, formulation, parameter or other quantity or characteristic is "about," "approximate," or "at or about" whether or not expressly stated to be such. It is understood that where "about," "approximate," or "at or about" is used before a quantitative value, the parameter also includes the specific quantitative value itself, unless specifically stated otherwise.

As used herein, the term "subject" can be a vertebrate, such as a mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. In an aspect, a mammalian subject is a human. The term "patient" includes human and veterinary subjects.

As used herein, the terms "administering" and "administration" refer to any method of providing a disclosed composition to a subject.

As used herein, the terms "comprises," "comprising," "includes," "including," "containing," "characterized by," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. The term "comprising" may also include the limitations associated with the use of "consisting of" or "consisting essentially of".

The transitional phrase "consisting of excludes any element, step, or ingredient not specified in the claim, closing the claim to the inclusion of materials other than those recited except for impurities ordinarily associated therewith. When the phrase "consists of appears in a clause of the body of a claim, rather than immediately following the preamble, it limits only the element set forth in that clause; other elements are not excluded from the claim as a whole.

The transitional phrase "consisting essentially of limits the scope of a claim to the specified materials or steps and those that do not materially affect the basic and novel characteristic(s) of the claimed invention. A 'consisting essentially of claim occupies a middle ground between closed claims that are written in a 'consisting of format and fully open claims that are drafted in a 'comprising' format. Optional additives as defined herein, at a level that is appropriate for such additives, and minor impurities are not excluded from a composition by the term "consisting essentially of'.

When a composition, a process, a structure, or a portion of a composition, a process, or a structure, is described herein using an open-ended term such as "comprising," unless otherwise stated the description also includes an embodiment that "consists essentially of or "consists of the elements of the composition, the process, the structure, or the portion of the composition, the process, or the structure.

The articles "a" and "an" may be employed in connection with various elements and components of compositions, processes or structures described herein. This is merely for convenience and to give a general sense of the compositions, processes or structures. Such a description includes "one or at least one" of the elements or components. Moreover, as used herein, the singular articles also include a description of a plurality of elements or components, unless it is apparent from a specific context that the plural is excluded.

The term "about" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. In general, an amount, size, formulation, parameter or other quantity or characteristic is "about" or "approximate" whether or not expressly stated to be such.

The term "or", as used herein, is inclusive; that is, the phrase "A or B" means "A, B, or both A and B". More specifically, a condition "A or B" is satisfied by any one of the following: A is true (or present) and B is false (or not present); A is false (or not present) and B is true (or present); or both A and B are true (or present). Exclusive "or" is designated herein by terms such as "either A or B" and "one of A or B", for example.

In addition, the ranges set forth herein include their endpoints unless expressly stated otherwise. Further, when an amount, concentration, or other value or parameter is given as a range, one or more preferred ranges or a list of upper preferable values and lower preferable values, this is to be understood as specifically disclosing all ranges formed from any pair of any upper range limit or preferred value and any lower range limit or preferred value, regardless of whether such pairs are separately disclosed. The scope of the invention is not limited to the specific values recited when defining a range.

When materials, methods, or machinery are described herein with the term "known to those of skill in the art", "conventional" or a synonymous word or phrase, the term signifies that materials, methods, and machinery that are conventional at the time of filing the present application are encompassed by this description. Also encompassed are materials, methods, and machinery that are not presently conventional, but that will have become recognized in the art as suitable for a similar purpose.

Unless stated otherwise, all percentages, parts, ratios, and like amounts, are defined by weight.

### EXAMPLES

### Example 1 Synthesis of benzotriazole conjugate 1

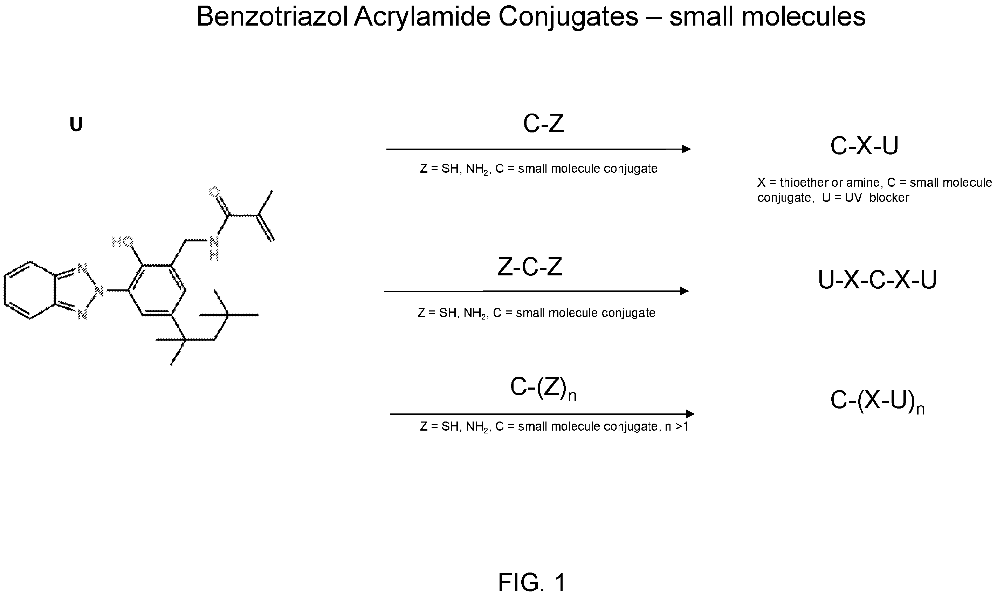

N-[[3-(benzotriazol-2-yl)-2-hydroxy-5-(2,4,4-trimethylpentan-2-yl)phenyl]methyl]-2-methylprop-2-enamide (CAS 107479-06-1) is added to chloroform. 1,6-hexanediamine is added to the chloroform in a molar ratio of 2.5:1 benzotriazol:diamine. The mixture is heated to about 40 °C for about 4 hrs. The resultant reaction mixture is concentrated using a rotavap. The conjugate is purified by silica gel column chromatography. As shown in FIG. 1, the N-[[3-(benzotriazol-2-yl)-2-hydroxy-5-(2,4,4-trimethylpentan-2-yl)phenyl]methyl]-2-methylprop-2-enamide (shown on the left hand side and is U), is reacted with the Z of 1,6-hexanediamine (C) to form the conjugate C-(X-U)ₙ where n = 2. This is reaction is exemplary for the other compounds shown in FIG. 1 wherein there are two or more Z moieties used in making the UV-absorbing conjugate compounds.

### Example 2 Synthesis of benzotriazole hydrazide

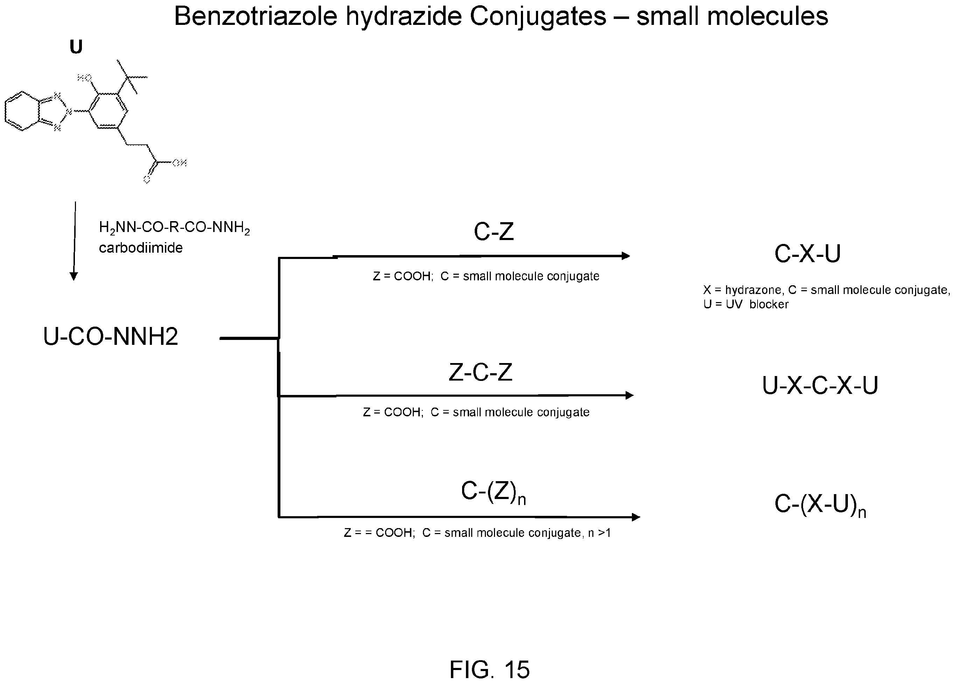

3-[3-(2H-Benzotriazol-2-yl)-5-tert-butyl-4-hydroxyphenyl]propionic acid (CAS 84268-36-0) is dissolved is a suitable organic solvent (for example dichloromethane, ethyl acetate, methyl ethyl ketone). Dicyclocarbodiimide (DCC) in about a 1:1 molar ratio is added to the solution and the solution is stirred for 5 minutes. An excess molar ratio of Adipic dihydrazide is added to the stirring solution. The reaction mixture is allowed to react for 4 hrs. The precipitate is removed by filtration. The solution is then extracted at least 4 times with acidified water in order to extract the remaining adipic dihydrazide. The resultant benzotriazole hydrazide is recrystallized from a suitable organic solvent. As shown in FIG. 15, the reaction of the 3-[3-(2H-Benzotriazol-2-yl)-5-tert-butyl-4-hydroxyphenyl]propionic acid with the dihydrazide forms the conjugate U-CO-NNH2.

### Example 3 Synthesis of benzotriazole-HA conjugate

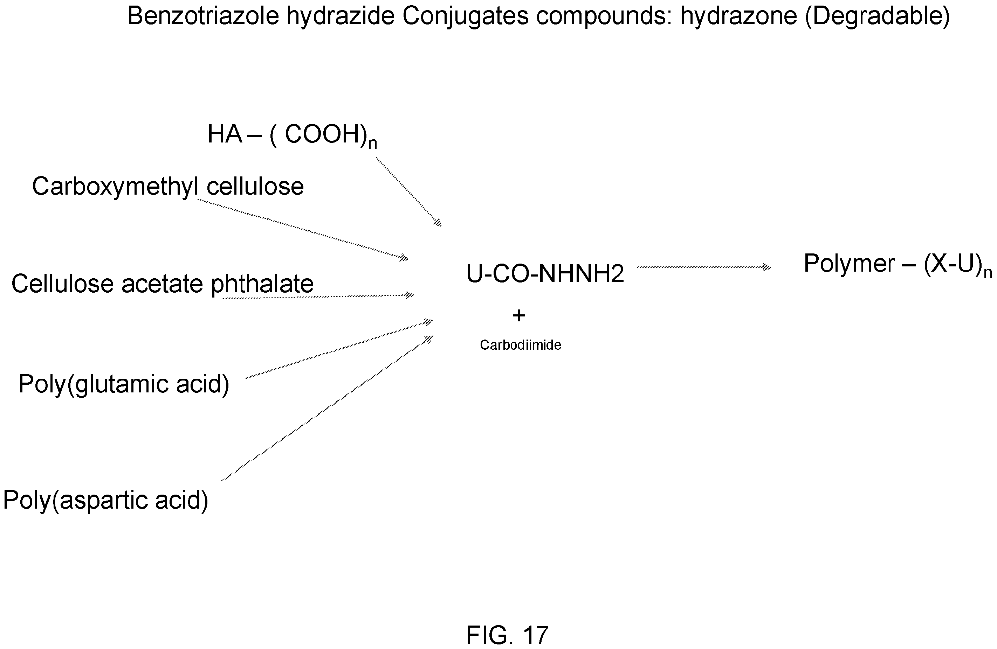

Sodium hyaluronate (50 kDa) was added to DMSO. The benzotriazole hydrazide from Example 2 is added to the DMSO mixture at a 0.1: 1 molar ratio of the hydrazide to the disaccharide units of the hyaluronic acid. Dicyclocarbodiimide (DCC) in about a 1:1 molar ratio to the hydrazide is added. The reaction mixture is allowed to stir overnight. Any formed precipitate was filtered and the resultant mixture is dialysed against water to remove the DMSO. Following dialysis, the contents of the dialysis bag are added to an excess cold ethanol. The precipitated material is filtered. The precipitate it titurated with methyl ethyl ketone. The benzatriazole-HA conjugate was dried under vacuum. As shown in FIG. 17, the benzotriazole hydrazide (shown as U-CO-NHNH2), is reacted with the carboxylic acid group of hyaluronic acid (C) to form the conjugate C-(X-U)ₙ where X is a -CO-NHNH-CO- group and n > 1. This is reaction is exemplary for the other compounds shown in FIG. 17 wherein other conjugate compounds that have multiple carboxylic acid groups are used in making the UV-absorbing conjugate compounds.

### Example 4 Synthesis of benzotriazole-Cellulose conjugate 1

1g cellulose acetate phthalate was dissolved in 50 mL Ethyl acetate : ethanol (1 : 1). Dicyclocarbodiimide (DCC) is added to the solution in a 0.9:1 molar ratio with the phthalate groups. The solution is stirred for 5 minutes after which the benzotriazole hydrazide from Example 2 is added to the solution in a 0.8:1 molar ratio of phthalate groups. The reaction mixture is allowed to stir overnight. Any formed precipitate was filtered from the solution. The resultant reaction mixture is concentrated using a rotavap. The solution is poured into an excess cold water to precipitate out the product. The precipitate is filtered and is washed with ethanol. The product is dried under vacuum.. As shown in FIG. 17, the benzotriazole hydrazide (shown as U-CO-NHNH2), is reacted with the carboxylic acid group of cellulose acetate phthalate (C) to form the conjugate C-(X-U)n where X is a -CO-NHNH-CO- group and n >1. This is reaction is exemplary for the other compounds shown in FIG. 17 wherein other conjugate compounds that have multiple carboxylic acid groups are used in making the UV-absorbing conjugate compounds.

### Example 5 Synthesis of benzotriazole - poly acrylic acid conjugate

Poly(acrylic acid, sodium salt) solution (average Mw ~1,200, 45 wt. % in H2O) is precipitated out of solution by adding 2M HCl dropwise. The precipitated poly(acrylic acid) is filtered and dried under vacuum. The poly(acrylic acid) is added to DMSO at 10% (w/v). Dicyclocarbodiimide (DCC) is added to the solution in a 0.9:1 molar ratio with the carboxylic acid groups. The solution is stirred for 5 minutes after which the benzotriazole hydrazide from Example 2 is added to the solution in a 0.8:1 molar ratio of carboxylic acid groups. The reaction mixture is allowed to stir overnight. Any formed precipitate was filtered from the solution. The resultant reaction mixture is concentrated using a rotavap. The solution is poured into an excess cold water to precipitate out the product. The precipitate is filtered and is washed with ethanol. The product is dried under vacuum.

### Example 6 Synthesis of partially deacetylated hyaluronic acid

300 mL of hydrazine monohydrate is added to 6 g of hyaluronic acid (50 kDa) for a 2% w/v polymer solution. 3 g hydrazine sulfate is added to this solution. The resultant solution is stirred at 55° C for 72-96 hours. 120 mL of cold ethanol is added to the reaction mixture to precipitate out the hyaluronic acid product. The precipitated material is filtered, washed with ethanol, and dried under vacuum for 24 hours.

The dried material is added to a beaker and 100 mL of acetic acid and 60 mL of 0.5M iodic acid (HIO₃) is added to the beaker. The solution stirred in a water bath at 4° C for at least 1 hour. An aqueous HI (57%, 17.5 mL) is added and the mixture is stirred for 15 minutes.

The solution is transferred to a separatory funnel to which 150 mL diethyl ether is added. The mixture is shaken vigorously and the aqueous layer recovered. The extraction is repeated with diethyl ether until the violet colour is no longer visible in the organic layer. The pH of the collected aqueous solution is adjusted to 7-7.5 with 0.2 M NaOH. The polymer is precipitated by adding excess cold ethanol to the solution. The precipitate is filtered, washed with ethanol and then dried under vacuum.

### Example 7 Synthesis of benzotriazole-HA conjugate 2

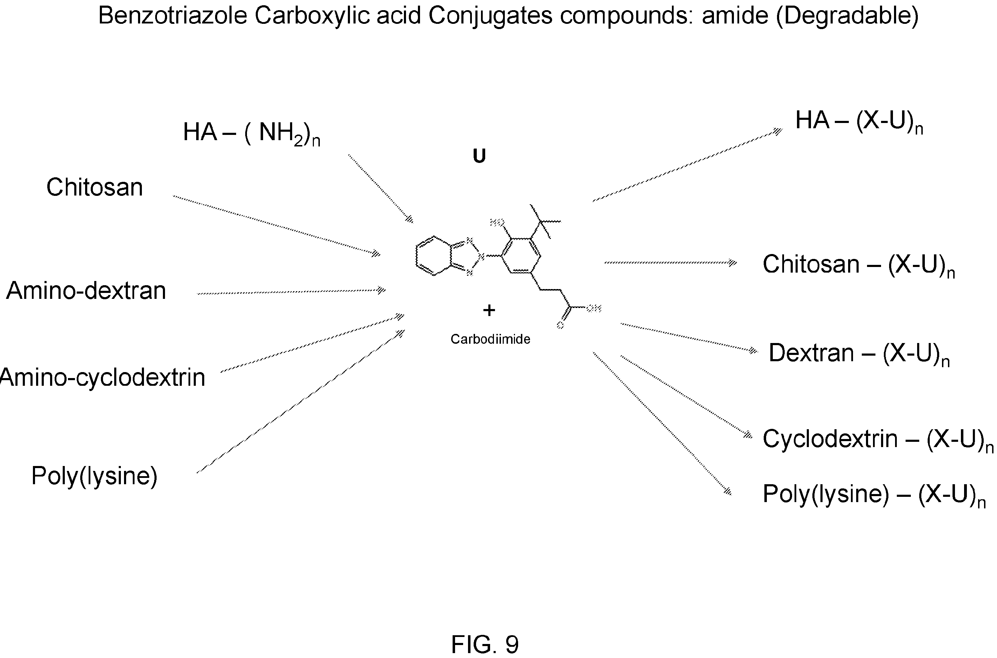

The of partially deacetylated hyaluronic acid (Example 6) was added to DMSO. 3-[3-(2H-Benzotriazol-2-yl)-5-tert-butyl-4-hydroxyphenyl]propionic acid (CAS 84268-36-0) is added to the DMSO mixture at a 1:1 molar ratio of the carboxylic acid to amine groups of the partially deacetylated hyaluronic acid. Dicyclocarbodiimide (DCC) in about a 1:1 molar ratio to the carboxylic acid groups is added. The reaction mixture is allowed to stir overnight. Any formed precipitate was filtered and the resultant mixture is dialysed against water to remove the DMSO. Following dialysis, the contents of the dialysis bag are added to an excess cold ethanol. The precipitated material is filtered. The precipitate it titurated with methyl ethyl ketone. The benzatriazole-HA conjugate was dried under vacuum. As shown in FIG. 9, the 3-[3-(2H-Benzotriazol-2-yl)-5-tert-butyl-4-hydroxyphenyl]propionic acid is reacted with the amine group of deacetylated hyaluronic acid (C) to form the conjugate C-(X-U)n where X is an amide and n > 1. This is reaction is exemplary for the other compounds shown in FIG. 9 wherein other conjugate compounds that have multiple amine groups are used in making the UV-absorbing conjugate compounds.

### Example 8 Synthesis of benzotriazole-PEG conjugate 1

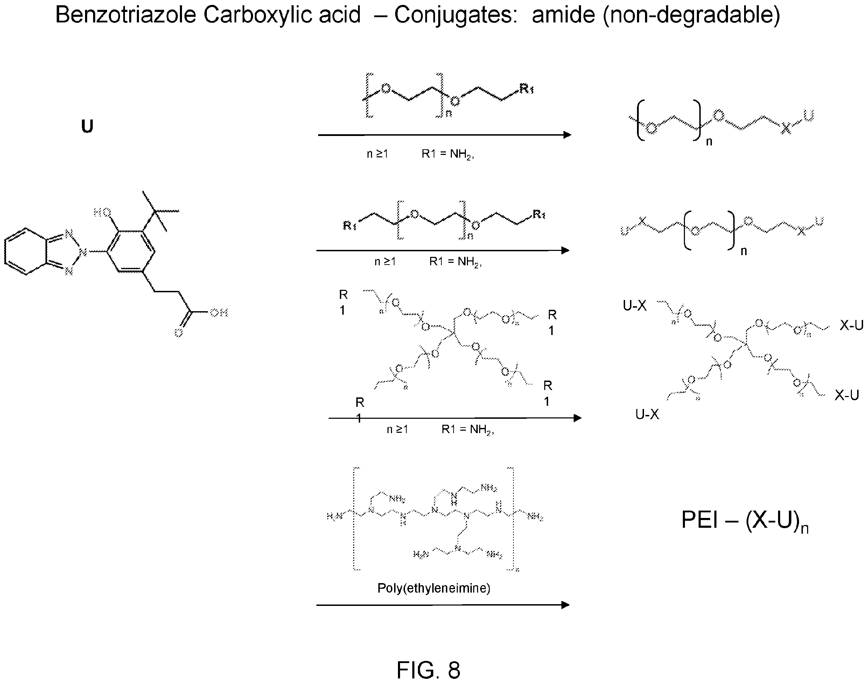

1g 3-[3-(2H-Benzotriazol-2-yl)-5-tert-butyl-4-hydroxyphenyl]propionic acid (CAS 84268-36-0) is added to dichloromethane. Dicyclocarbodiimide (DCC) is added to the dichloromethane in a 1:1 Molar ratio. The solution is stirred for 5 minutes after which PEG-diamine (2,000; Sigma-Aldrich) is added to the dichloromethane in a 1.2:1 molar ratio of carboxylic acid groups to PEG amine groups. The reaction mixture is allowed to stir overnight. Any formed precipitate was filtered from the solution. The resultant reaction mixture is concentrated using a rotavap. The conjugate is purified by silica gel column chromatography. As shown in FIG. 8, the 3-[3-(2H-Benzotriazol-2-yl)-5-tert-butyl-4-hydroxyphenyl]propionic acid is reacted with the amine groups of PEG-diamine (C) to form the conjugate C-(X-U)n where n =2 and X is an amide. This is reaction is exemplary for the other compounds shown in FIG. 8 wherein other conjugate compounds that have at least one amine groups are used in making the UV-absorbing conjugate compounds.

### Example 9 Synthesis of benzotriazole-cyclodextrin conjugate 1

1g 3-[3-(2H-Benzotriazol-2-yl)-5-tert-butyl-4-hydroxyphenyl]propionic acid (CAS 84268-36-0) is added to DMSO. Dicyclocarbodiimide (DCC) is added to the DMSO in a 1:1 Molar ratio. The solution is stirred for 5 minutes after which 6-Monoamino-6-monodeoxy-beta-Cyclodextrin hydrochloride is added to the DMSO in a 1.2:1 molar ratio of carboxylic acid groups to PEG amine groups. The reaction mixture is allowed to stir overnight. Any formed precipitate was filtered from the solution. The resultant reaction mixture is concentrated using a rotavap. The conjugate is purified by silica gel column chromatography. As shown in FIG. 9, the 3-[3-(2H-Benzotriazol-2-yl)-5-tert-butyl-4-hydroxyphenyl]propionic acid is reacted with the amine groups of amino-cyclodextrin (C) to form the conjugate C-(X-U)n where n =1 and X is an amide. This is reaction is exemplary for the other compounds shown in FIG. 9 wherein other conjugate compounds that have at least one amine groups are used in making the UV-absorbing conjugate compounds.

### Example 10 Synthesis of benzophenone 4 -hexane conjugate

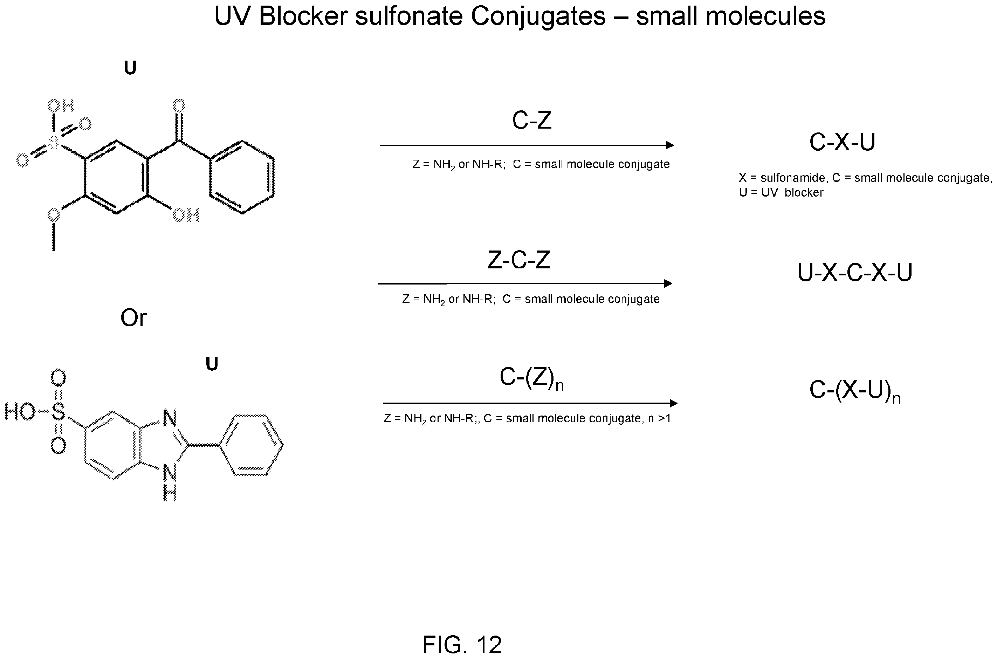

1g Benzophenone-4 (CAS 4065-45-6) is added to 50 mL anhydrous dimethyl formamide (DMF). Thionyl chloride is added to the reaction mixture such that the molar ratio of thionyl chloride: sulfonic acid groups is 0.95: 1. The mixture was stirred for 4 hrs after which and excess (1.2: 1 amine groups to sulfonic acid groups) of 1,6-hexane diamine was added. The reaction is heated to 50°C and run overnight. The reaction mixture is poured into excess water. The precipitate is filtered, washed with water and then dried under vacuum. As shown in FIG. 12, the benzophenone-4 is reacted with the amine groups (Z) of hexane diamine (C) to form the conjugate C-(X-U)n where n =2 and X is a sulfonamide or U-X-C-X-U. This is reaction is exemplary for the other compounds shown in FIG. 12 wherein other conjugate compounds that have at least one amine groups are used in making the UV-absorbing conjugate compounds.

### Example 11 Synthesis of UV Absorbing Polymer

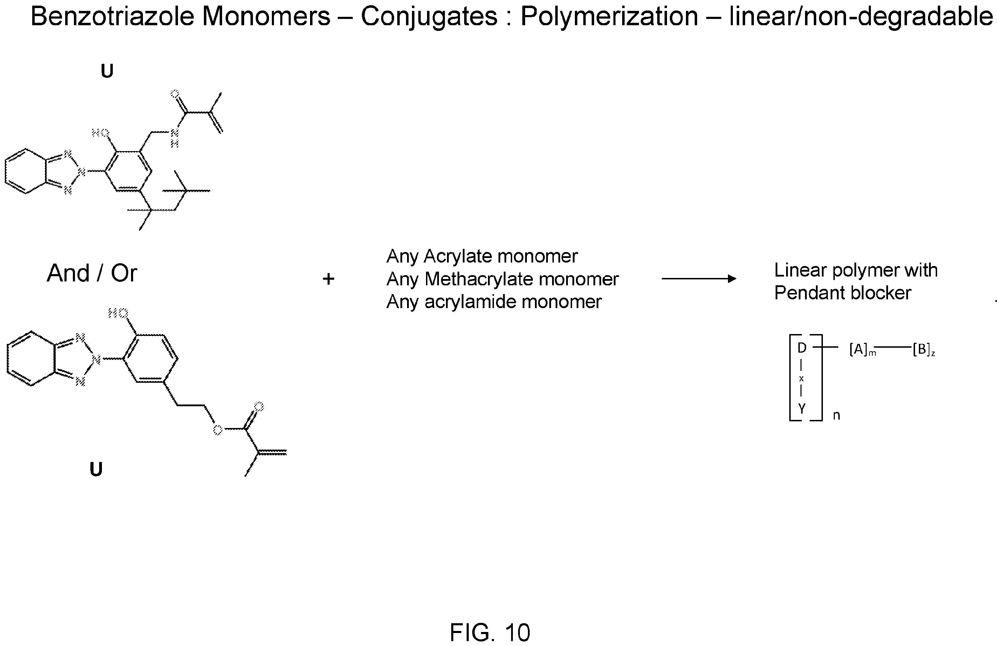

1g N-[[3-(benzotriazol-2-yl)-2-hydroxy-5-(2,4,4-trimethylpentan-2-yl)phenyl]methyl]-2-methylprop-2-enamide and 2 g 2-Hydroxyethyl methacrylate are added to 50mL dimethylsulfoxide. 300 mg AIBN is added to the mixture. The mixture is bubbled with nitrogen for 15 min. The mixture is heated to 60°C for about 18 hrs. The mixture is added to water to precipitate the formed polymer. The polymer is dried under vacuum. As shown in FIG. 10, the N-[[3-(benzotriazol-2-yl)-2-hydroxy-5-(2,4,4-trimethylpentan-2-yl)phenyl]methyl]-2-methylprop-2-enamide polymerized with 2-hydroxyethyl methacrylate (A) to form a polymer as shown as 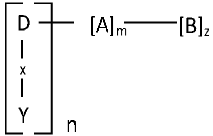 where D-X-Y is the reaction residue of the N-[[3-(benzotriazol-2-yl)-2-hydroxy-5-(2,4,4-trimethylpentan-2-yl)phenyl]methyl]-2-methylprop-2-enamide, n > 1, m > 1 and z = 0. This is reaction is exemplary for the other compounds shown in FIG. 10 wherein other monomers are used in making the UV-absorbing conjugate compounds.

### Example 12 UV absorbing conjugate particles

1 g hyaluronic acid is dissolved in 10 mL 0.25M NaOH. Once dissolved, 0.06 g 1,4-butanediol diglycidyl ether (BDDE) is added. The mixture is heated to 50°C for 3 hrs. The formed gel is added to 500 mL water and 0.2 M HCl is added to neutralize the solution. The supernatant water is decanted and the washing step is repeated two additional times. The gel is then added to a 60 mL syringe. The gel is forced through a 100 mesh screen contained in a filter holder. The gel is then forced through a 400 mesh screen contained in a filter holder. The gel is then dried under vacuum. The dried gel particles are added to 50 mL DMSO. 0.1g benzotriazole hydrazide compound (Example 2) is added to the reaction mixture. EDC (0.9:1 EDC to hydrazide molar ratio) is added to the reaction mixture. The mixture is allowed to react overnight. The mixture is poured into excess water. The gel particles are filtered and dried under vacuum. The dried particles are washed with ethyl acetate and are then dried under vacuum.

### Example 13 Sunscreen 1

Three different sunscreen formulations are prepared using the ingredients shown in Table 1 below.

**Table 1**

| | | Percent (w/w) | | |
|---|---|---|---|---|
| Phase | Ingredient | F1 | F2 | F3 |
| A | Water | 54.9 | 52.9 | 50.9 |
| | sclerotium gum | 0.3 | 0.3 | 0.3 |
| | Dekaben C-4 (Phenoxyethanol / Methylparaben/Ethylparaben/ Butylparaben/Propylparaben) | 1 | 1 | 1 |
| | Permulen TR-2 | 0.3 | 0.3 | 0.3 |
| B | UV absorbing conjugate | 15 | 15 | 15 |
| | Dicaprylyl Carbonate | 22.5 | 22.5 | 22.5 |
| | cetyl phosphate - potassium salt | 6 | 6 | 6 |
| | cetyl alcohol | 0 | 2 | 4 |

The water is added to the container and is heated to about 75 to 80°C. The remaining Phase A ingredients are added and mixed with a dispersion mixer until dissolved. The Phase B ingredients are added together and heated with mixing to about 75 to 80°C. Phase A mixture is added to phase B while mixing with a homogenizer. Under gentle stirring, the resultant emulsion is cooled to room temperature using a water bath. The formulation is prepared separately using UV absorbing conjugate as prepared in examples 1, 3-5 and 7-12.

### Example 14 Sunscreen 2

**Table 2**

| | | % (w/w) | | | |
|---|---|---|---|---|---|
| Part | Ingredient | F4 | F5 | F6 | F7 |
| A | water | 51.7 | 51.7 | 51.7 | 51.7 |
| | glycerin | 3.0 | 3.0 | 3.0 | 3.0 |
| | Xanthan gum | 0.2 | 0.2 | 0.2 | 0.2 |
| | trisodium ethylenediamine disuccinate | 0.3 | 0.3 | 0.3 | 0.3 |
| | Oliven 1000 4 (cetearl olivate/sorbitan olivate) | 6.0 | 6.0 | 6.0 | 6.0 |
| | Oliwax LC 4 (Cetyl palmitate/ sorbitan olivate) | 2.0 | 2.0 | 2.0 | 2.0 |
| | UV absorbing conjugate (Example 4) | 19.2 | 0.0 | 0.0 | 0.0 |
| | UV absorbing conjugate (Example 5) | 0.0 | 19.2 | 0.0 | 0.0 |
| B | UV absorbing conjugate (Example 6) | 0.0 | 0.0 | 19.2 | 0.0 |
| | UV absorbing conjugate (Example 3) | 0.0 | 0.0 | 0.0 | 19.2 |
| | ethylhexyl methoxycrylene | 4.0 | 4.0 | 4.0 | 4.0 |
| | c12-15 alkyl benzoate | 5.0 | 5.0 | 5.0 | 5.0 |
| | Caprylic/capric triglyceride | 5.0 | 5.0 | 5.0 | 5.0 |
| | CoSept PEP 7 (phenoxyethanol/ methylparaben/ ethylparaben/ butylparaben/ propylparaben/ isobutylparaben | 0.5 | 0.5 | 0.5 | 0.5 |
| | BHT | 0.1 | 0.1 | 0.1 | 0.1 |
| C | cyclopentasiloxane | 3.0 | 3.0 | 3.0 | 3.0 |

The glycerin and xanthan gum are added together and mixer with a dispersion mixer. The water is then added to the mixture with dispersion mixing. The mixture is heated to about 70 to 75°C. The Phase B ingredients are added together and heated with mixing to about 70 to 75°C. Phase B is added to phase A while mixing with a homogenizer. Under gentle stirring, the resultant emulsion is cooled to about 40°C using a water bath. Phase C is added to the emulsion and the mixture is homogenized for 2-5 minutes. The formulation is then cooled to room temperature while stirring gently.

### Example 15 Sunscreen 3

**Table 3**

| Phase | Ingredient | % (w/w) |
|---|---|---|
| A | Water | 45.2 |
| | Dermofeel^{®} PA-3 (Sodium Phytate/Water/Alcohol) | 0.1 |
| | Xanthan Gum | 0.2 |
| | Chlorphenesin | 0.3 |
| B | Heligogel (Sodium Acrylates Copolymer/Hydrogenated Polydecene/phospholipids/ Polyglyceryl-10 Stearate/ Helianthus Annuus (Sunflower) Seed Oil | 2.0 |
| | Heliofeel (Glyceryl Stearate Citrate/Polyglyceryl-3 Stearate /Hydrogenated Lecithin) | 5.0 |
| | Dekaben C-4 (Phenoxyethanol / Methylparaben/Ethylparaben/ Butylparaben/Propylparaben | 0.8 |
| | UV absorbing conjugate | 30 |
| | Vitapherole^{®}E-1000 (Tocopherol/Helianthus Annuus (Sunflower) Seed Oil) | 0.2 |
| | Butylene Glycol Dicaprvlate/ Dicaprate | 4.0 |
| | Dimethicone | 1.0 |
| | C12-C15 Alkyl Benzoate | 5.0 |
| | Dicaprylyl Carbonate | 5.0 |
| C | Melitane^{™} (Glycerin/Water/ Dextran/Acetyl Hexapeptide-1 | 0.5 |

The Dermofeel^{®} PA-3 , xanthan gum and chlorphenesin were added together. The water was then added and under high shear mixing, the mixture was heated to about 80-85°C. In a separate beaker, the ingredients for Phase B were added together and the mixture was heated to about 80-85°C. Part B is then added to Part A under high shear stirring. The mixture is stirred for about 3 - 5 minutes. The resultant mixture is allowed to cool under medium stirring. The Melitane is added once the temperature is below 40 °C. The mixture is allowed to cool to room temperature and, if necessary, the pH is adjusted to pH 5.0 to 5.5. The formulation is prepared separately using UV absorbing conjugate as prepared in examples 1, 3-5 and 7-12.

### Example 16

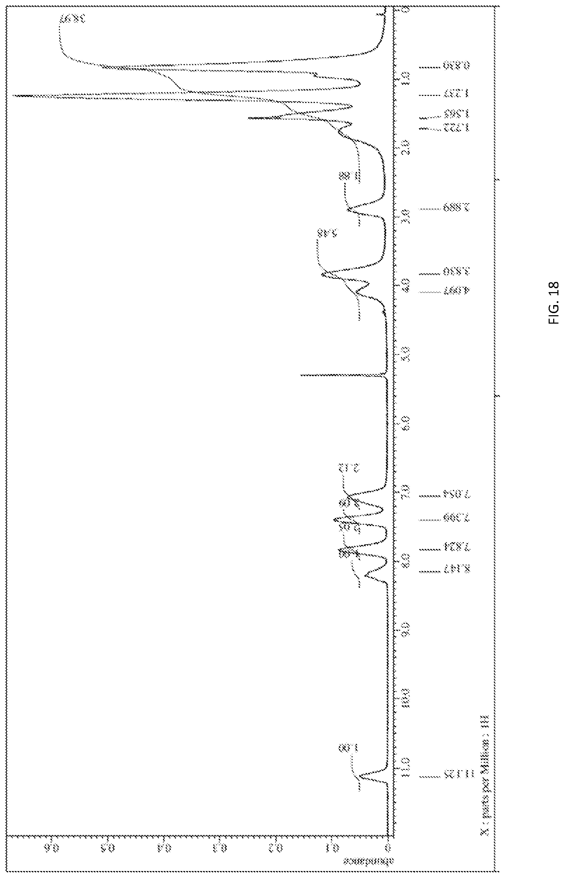

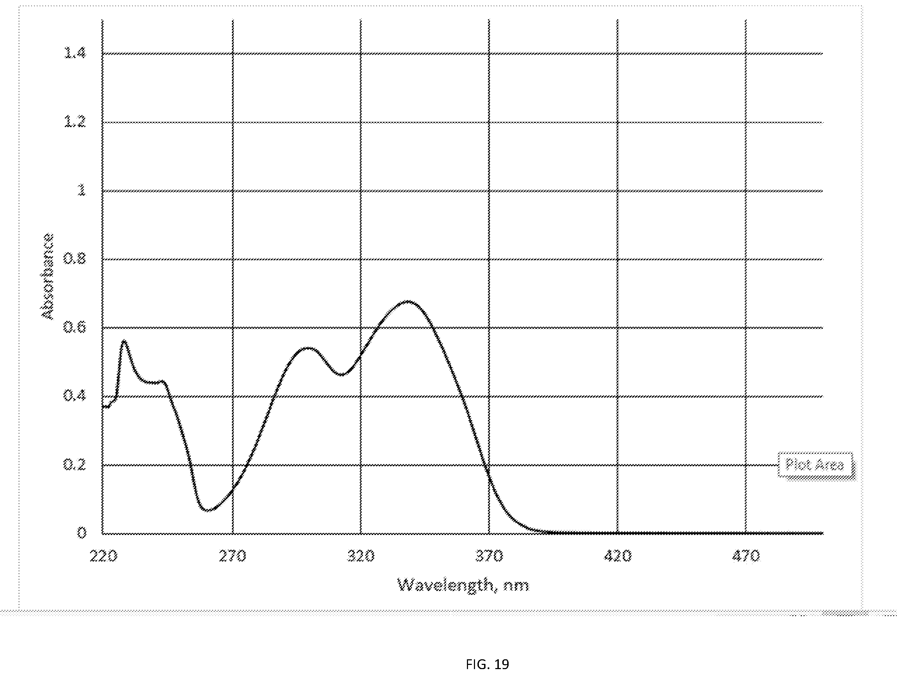

1g sodium dodecyl sulfate was added to 100 g deionized water. 200 mg potassium persulfate was added to the solution. The solution was stirred at about 250-280 rpm. The solution was heated to about 40°C and stirred until the potassium persulfate had dissolved. The solution was degassed with a stream of nitrogen. 4.92g 2-[2-Hydroxy-5-[2-(methacryloyloxy)ethyl]phenyl]-2H-benzotriazole and 5.18g hexylmethacrylate were added to 8.55g toluene. The suspension was vortexed and poured into the water solution. Under a flow of nitrogen, the solution was heated to about 70°C. The solution was stirred for about 3 hours. The solution was cooled to room temperature and filtered. The sample was dried under vacuum. The NMR is shown in Fig. 18. The UV spectrum of the material in dichloromethane is shown in Fig. 19.

### Example 17 Synthesis of acid chloride derivative

The glassware for the reaction was dried overnight in an oven. 25.1 g 3-[3-(benzotriazol-2-yl)-5-tert-butyl-4-hydroxyphenyl]propanoic acid,(CAS 84268-36-0) was added to a round bottom flask. 249 mL anhydrous toluene was added to the flask using a cannula and nitrogen. 1.5 mL anhydrous DMF was added to the reaction mixture. The reaction mixture was stirred at about 400 rpm. 8.33 mL thionyl chloride was added to the reaction mixture using a syringe and a 18G needle.

The reaction mixture was heated to 80°C for 4 hours. The reaction mixture was cooled to room temperature and the toluene was removed under vacuum. Approximately 126 mL hexane was added to the residue. The residue was left overnight and the supernatant was decanted off. The precipitated material was triturated with about 60 mL hexane. This was repeated five times. The sample was dried under vacuum.

### Example 18 Chitosan-benzotriazole derivative

4 g Chitosan oligosaccharide (TCI) was dried under vacuum at 50 °C. 102g anhydrous DMF was added to the chitosan. About 2.2 mL triethylamine was added to the reaction mixture. The mixture was stirred at about 400 rpm. 4 g of the sulphonyl chloride from Example 2 was added to the reaction mixture. The temperature was increased to 80°C and the mixture was stirred at 200 rpm overnight. The reaction mixture was cooled to room temperature.

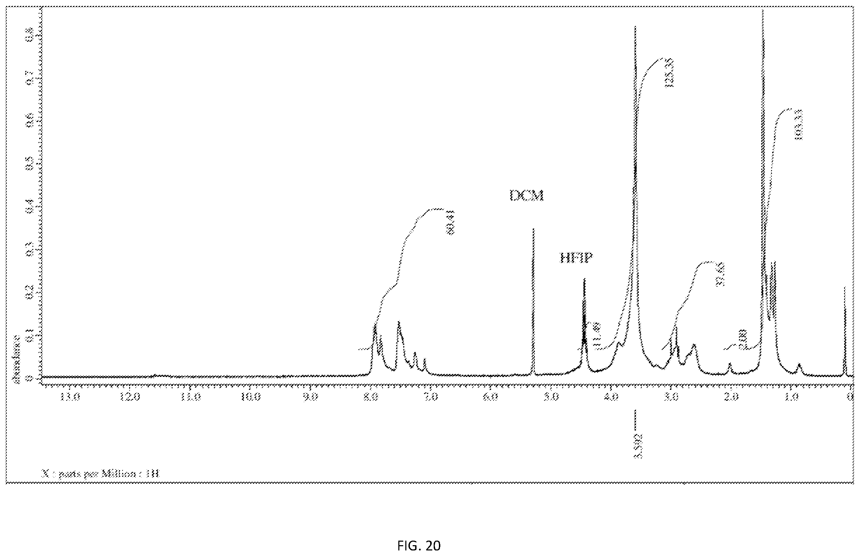

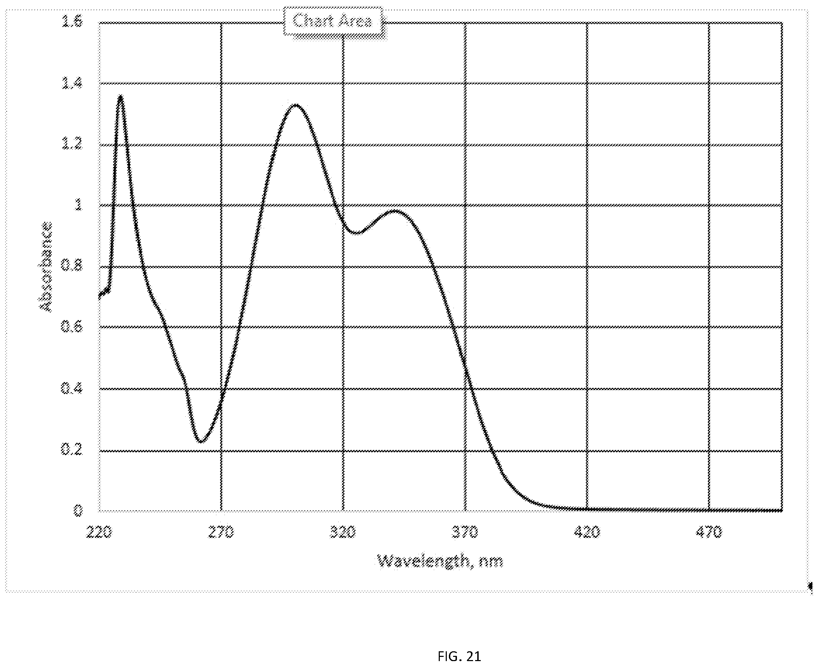

About 200 mL water (pH 8.3) was added to the reaction mixture. The solution was stirred until a precipitate formed. The precipitate was centrifuges and the supernatant was decanted off. This process was repeated 4 times. A water/methanol (60/40 v/v) was added to the precipitate to wash the precipitate. After centrifugation, the supernatant was decanted. This process was repeated with methanol. The precipitate was dried under vacuum. The NMR is shown in Figure 20. The UV spectrum of the material in dichloromethane is shown in Figure 21.

## Claims

1. A topical formulation composition comprising one or more UV absorbing conjugate compounds comprising a UV absorbing compound conjugated with a conjugate compound, wherein the a UV absorbing compound is selected from a group consisting of a benzophenone compound conjugated to a conjugate compound via a sulfonamide linker wherein the benzophenone conjugate compound has a molecular weight of at least 800 daltons; a benzimidazole compound conjugated to a conjugate compound via a sulfonamide linker wherein the benzimidazole conjugate compound has a molecular weight of at least 800 daltons; a benzotriazole compound conjugated to a conjugate compound via an amide linker wherein the benzotriazole conjugate compound has a molecular weight of at least 800 daltons; a benzotriazole compound conjugated to a conjugate compound through the product of a Michael addition reaction wherein the benzotriazole conjugate compound has a molecular weight of at least 800 daltons; a benzotriazole functionalized polymer wherein benzotriazole functionalized polymer has a molecular weight of at least 800 daltons and wherein the benzotriazole is bonded to the polymer as a methacrylate, acrylate or acrylamide derivative of a benzotriazole compound; and a benzotriazole compound conjugated to a polysaccharide wherein the benzotriazole polysaccharide conjugate compound has a molecular weight of at least 800 daltons, wherein the UV absorbing conjugate compound is not transported or absorbed through the skin.

2. The composition of claim 1, wherein the UV absorbing compound comprises
(U -X)n-C,
wherein, U is an UV absorbing compound moiety, X is a thioether, amine, amide, urethane, sulfonamide group, or -CO-NH-NH-CO-, C is a conjugate compound moiety and n is an integer and where n is ≥1.

3. The composition of claim 1, wherein the UV absorbing compound comprises
(U -X)n-C,
wherein, U is an UV absorbing compound moiety, X is an ester, C is a conjugate compound moiety and n is an integer and where n is ≥1.

4. The composition of claim 1, wherein the UV absorbing conjugate compound has the structure, 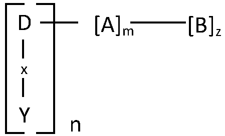 wherein Y is an UV absorbing compound moiety, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n is an integer and where n is ≥1 and m and z are each an integer and where m is ≥0, and z is ≥0.

5. The composition of claim 4 wherein the UV absorbing conjugate compound is a block copolymer or a random copolymer.

6. The composition of claim 1, wherein the UV absorbing conjugate compound comprises
(U -X)n -C-(X-U1)m,
wherein, U is an UV absorbing compound moiety, U1 is a UV absorbing compound moiety that has a different chemical structure to U, X is a thioether, amine, amide, ester, urethane, sulfonamide group, -CO-NH-NH-CO-, C is a conjugate compound moiety and n and m are each an integer and where n and m is ≥1.

7. The composition of claim 1, wherein the UV absorbing conjugate compound comprises, 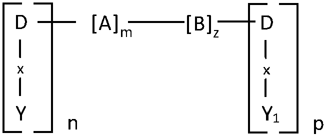 wherein Y is an UV absorbing compound moiety, Y1 is a UV absorbing compound moiety that has a different chemical structure to Y, X is a thioether, amine, amide, ester, urethane, sulfonamide group, -CO-NH-NH-CO-, D is a residue of a vinyl, acrylate, methacrylate or acrylamide group, A is a residue of a vinyl, acrylate, methacrylate or acrylamide monomer, B is a residue of a vinyl, acrylate, methacrylate or acrylamide monomer that is different from A, n and p are each an integer and where n and p is ≥1 and m and z are each an integer and where m is ≥0, and z is ≥0. The structure in Formula (4) can be a block copolymer or it can be a random copolymer.

8. The composition of claim 7 wherein the UV absorbing conjugate compound is a block copolymer or a random copolymer.

9. The composition of claim 1, wherein UV absorbing conjugate compounds comprise benzophenone-based compounds, benzotriazole-based compounds, or benzimidazole-based compounds that have absorbance from about 200 to about 380 nm range.

10. The composition of claim 9, wherein the UV absorbing conjugate compounds comprise one or more UV absorbing benzotriazole compounds comprising the reaction residues of an acrylamide group, acrylate group, methacrylate group, maleimide group, acrylonitrile groups or a vinyl sulfone group.

11. The topical formulation composition of claim 1, formulated as a sunscreen formulation comprising a daily use sunscreen, a water resistant sunscreen, or combinations thereof; a cosmetic formulation comprising a moisturizer, foundation, lip stick, lip gloss, chap stick, concealer, highlighter, a blush, eye shadows, cleansers, toners, serums, anti-aging products, setting sprays or combinations thereof; a hair product composition include, comprising, a shampoo, a conditioner, a leave-in conditioner, a hair mousse, a hair gel, a hair spray, a curling cream, a hair wax, a treatment oil, a medicated hair treatment or combinations thereof.

## Patentansprüche

1. Topische Formulierungszusammensetzung, die eine oder mehrere UV-absorbierende Konjugatverbindungen umfasst, die eine UV-absorbierende Verbindung enthält, die mit einer Konjugatverbindung konjugiert ist, wobei die UV-absorbierende Verbindung ausgewählt ist aus einer Gruppe bestehend aus einer Benzophenonverbindung, die über einen Sulfonamid-Linker mit einer Konjugatverbindung konjugiert ist, wobei die Benzophenonkonjugatverbindung ein Molekulargewicht von mindestens 800 Dalton aufweist, einer Benzimidazolverbindung, die über einen Sulfonamid-Linker mit einer Konjugatverbindung konjugiert ist, wobei die Benzimidazolkonjugatverbindung ein Molekulargewicht von 800 Dalton aufweist, einer Benzotriazolverbindung, die über einen Amid-Linker mit einer Konjugatverbindung konjugiert ist, wobei die Benzotriazolkonjugatverbindung ein Molekulargewicht von mindestens 800 Dalton aufweist, einer Benzotriazolverbindung, die über das Produkt einer Michael-Additionsreaktion mit einer Konjugatverbindung konjugiert ist, wobei die Benzotriazolkonjugatverbindung ein Molekulargewicht von mindestens 800 Dalton aufweist, einem Benzotriazol-funktionalisierten Polymer, wobei das Benzotriazolfunktionalisierte Polymer ein Molekulargewicht von mindestens 800 Dalton aufweist und wobei das Benzotriazol als Methacrylat-, Acrylat,- oder Acrylamid-Derivat einer Benzotriazolverbindung an das Polymer gebunden ist, und einer Benzotriazolverbindung, die an ein Polysaccharid konjugiert ist, wobei die Benzotriazolpolysaccharidkonjugatverbindung ein Molekulargewicht von mindestens 800 Dalton aufweist, wobei die UV-absorbierende Konjugatverbindung nicht durch die Haut transportiert oder absorbiert wird.

2. Zusammensetzung nach Anspruch 1, wobei die UV-absorbierende Verbindung
(U-X)ₙ-C umfasst,
wobei U eine UV-absorbierende Verbindungseinheit ist, X eine Thioether-, Amin-, Amid-, Urethan-, Sulfonamidgruppe oder -CO-NH-NH-CO- ist, C eine konjugierte Verbindungseinheit ist und n eine ganze Zahl ist, wobei n ≥1 ist.

3. Zusammensetzung nach Anspruch 1, wobei die UV-absorbierende Verbindung
(U-X)ₙ-C umfasst,
wobei U eine UV-absorbierender Verbindungseinheit ist, X ein Ester ist, C eine konjugierte Verbindungseinheit ist und n eine ganze Zahl ist, wobei n ≥1 ist.

4. Zusammensetzung nach Anspruch 1, wobei die UV-absorbierende Konjugatverbindung die Struktur 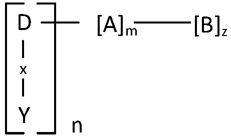 aufweist,
wobei Y eine UV-absorbierende Verbindungseinheit ist, X eine Thioether-, Amin-, Amid-, Ester-, Urethan-, Sulfonamidgruppe oder -CO-NH-NH-CO- ist, D ein Rest einer Vinyl-, Acrylat-, Methacrylat- oder Acrylamidgruppe ist, A ein Rest eines Vinyl-, Acrylat-, Methacrylat- oder Acrylamidmonomers ist, B ein Rest eines Vinyl-, Acrylat-, Methacrylat- oder Acrylamidmonomers ist, der von A verschieden ist, n eine ganze Zahl ist und wobei n ≥1 ist und m und z jeweils eine ganze Zahl sind, wobei m ≥0 ist und z ≥0 ist.

5. Zusammensetzung nach Anspruch 4, wobei die UV-absorbierende konjugierte Verbindung ein Blockcopolymer oder ein statistisches Copolymer ist.

6. Zusammensetzung nach Anspruch 1, wobei die UV-absorbierende Konjugatverbindung (U-X)ₙ-C-(X-U1)ₘ umfasst,
wobei U eine UV-absorbierende Verbindungseinheit ist, U1 eine UV-absorbierende Verbindungseinheit ist, die eine andere chemische Struktur als U hat, X eine Thioether-, Amin-, Amid-, Ester-, Urethan-, Sulfonamidgruppe oder -CO-NH-NH-CO- ist, C eine konjugierte Verbindungseinheit ist und n und m jeweils eine ganze Zahl sind, wobei n und m ≥1 ist.

7. Zusammensetzung nach Anspruch 1, wobei die UV-absorbierende Konjugatverbindung 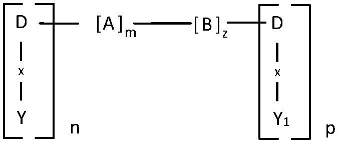 umfasst, wobei Y eine UV-absorbierende Verbindungseinheit ist, Y1 eine UV-absorbierende Verbindungseinheit ist, die eine andere chemische Struktur als Y hat, X eine Thioether-, Amin-, Amid-, Ester-, Urethan-, Sulfonamidgruppe oder -CO-NH-NH-CO- ist, D ein Rest einer Vinyl-, Acrylat-, Methacrylat- oder Acrylamidgruppe ist, A ein Rest eines Vinyl-, Acrylat-, Methacrylat- oder Acrylamidmonomers ist, B ein Rest eines Vinyl-, Acrylat-, Methacrylat- oder Acrylamidmonomers ist, der von A verschieden ist, n und p jeweils eine ganze Zahl sind und wobei n und p ≥1 ist und m und z jeweils eine ganze Zahl sind und wobei m ≥0 ist und z ≥0 ist, wobei die Struktur in Formel (4) ein Blockcopolymer oder ein statistisches Copolymer sein kann.

8. Zusammensetzung nach Anspruch 7, wobei die UV-absorbierende Konjugatverbindung ein Blockcopolymer oder ein statistisches Copolymer ist.

9. Zusammensetzung nach Anspruch 1, wobei die UV-absorbierenden Konjugatverbindungen Verbindungen auf Benzophenonbasis, Verbindungen auf Benzotriazolbasis oder Verbindungen auf Benzimidazolbasis umfassen, die eine Absorption im Bereich von etwa 200 bis etwa 380 nm aufweisen.

10. Zusammensetzung nach Anspruch 9, wobei die UV-absorbierenden Konjugatverbindungen eine oder mehrere UV-absorbierende Benzotriazolverbindungen umfassen, die die Reaktionsreste einer Acrylamidgruppe, Acrylatgruppe, Methacrylatgruppe, Maleinimidgruppe, Acrylnitrilgruppen oder einer Vinylsulfongruppe umfassen.

11. Topische Formulierungszusammensetzung nach Anspruch 1, formuliert als eine Sonnenschutzformulierung, die einen Sonnenschutz für den täglichen Gebrauch, einen wasserfesten Sonnenschutz oder Kombinationen davon umfasst; eine kosmetische Formulierung, die eine Feuchtigkeitscreme, eine Grundierung, einen Lippenstift, einen Lipgloss, einen Lippenbalsam, einen Verdecker, einen Highlighter, ein Rouge, einen Lidschatten, Reinigungsmittel, Toner, Seren, Anti-Aging-Produkte, Fixiersprays oder Kombinationen davon umfasst; oder eine Haarproduktzusammensetzung, die ein Shampoo, einen Conditioner, einen Leave-in-Conditioner, einen Haarschaum, ein Haargel, ein Haarspray, eine Lockencreme, ein Haarwachs, ein Behandlungsöl, eine medizinische Haarbehandlung oder Kombinationen davon umfasst.

## Revendications

1. Composition de formulation topique comprenant un ou plusieurs composés conjugués absorbant les UV comprenant un composé absorbant les UV conjugué à un composé conjugué, dans laquelle le composé absorbant les UV est choisi dans un groupe comprenant un composé à base de benzophénone conjugué à un composé conjugué par l'intermédiaire d'une liaison sulfonamide dans lequel le composé conjugué à base de benzophénone présente une masse moléculaire d'au moins 800 daltons ; un composé à base de benzimidazole conjugué à un composé conjugué par l'intermédiaire d'une liaison sulfonamide dans lequel le composé conjugué à base de benzimidazole présente une masse moléculaire d'au moins 800 daltons ; un composé à base de benzotriazole conjugué à un composé conjugué par l'intermédiaire d'une liaison amide dans lequel le composé conjugué à base de benzotriazole présente une masse moléculaire d'au moins 800 daltons ; un composé à base de benzotriazole conjugué à un composé conjugué à l'aide du produit d'une réaction d'addition de Michael dans lequel le composé conjugué à base de benzotriazole présente une masse moléculaire d'au moins 800 daltons ; un polymère fonctionnalisé à base de benzotriazole dans lequel le polymère fonctionnalisé à base de benzotriazole présente une masse moléculaire d'au moins 800 daltons et dans lequel le benzotriazole est lié au polymère en tant que dérivé de type méthacrylate, acrylate ou acrylaminde d'un composé à base de benzotriazole ; et un composé à base de benzotriazole conjugué à un polysaccharide dans lequel le composé conjugué à base de benzotriazole et de polysaccharide présente une masse moléculaire d'au moins 800 daltons, dans laquelle le composé conjugué absorbant les UV n'est pas transporté ni absorbé par la peau.

2. Composition selon la revendication 1, dans laquelle le composé absorbant les UV comprend un composé de type (U-X)n-C,
dans lequel, U représente un fragment de composé absorbant les UV, X représente un groupe thioéther, un groupe amine, un groupe amide, un groupe uréthane, un groupe sulfonamide, ou un groupe -CO-NH-NH-CO-, C représente un fragment de composé conjugué et n représente un nombre entier et dans lequel n est ≥ 1.

3. Composition selon la revendication 1, dans laquelle le composé absorbant les UV comprend un composé de type (U-X)n-C,
dans lequel, U représente un fragment de composé absorbant les UV, X représente un groupe ester, C représente un fragment de composé conjugué et n représente un nombre entier et dans lequel n est ≥ 1.

4. Composition selon la revendication 1, dans laquelle le composé conjugué absorbant les UV présente la structure, 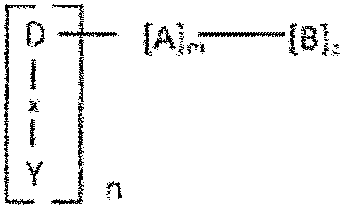 dans laquelle Y représente un fragment de composé absorbant les UV, X représente un groupe thioéther, un groupe amine, un groupe amide, un groupe ester, un groupe uréthane, un groupe sulfonamide, un groupe -CO-NH-NH-CO-, D représente un résidu d'un groupe vinyle, acrylate, méthacrylate ou acrylamide, A représente un résidu d'un groupe vinyle, acrylate, méthacrylate ou acrylamide, B représente un résidu d'un monomère de type vinyle, acrylate, méthacrylate ou acrylamide qui est différent de A, n représente un nombre entier et dans laquelle n est ≥ 1 et m et z représentent chacun un nombre entier et dans laquelle m est ≥ 0, et z est ≥ 0.

5. Composition selon la revendication 4 dans laquelle le composé conjugué absorbant les UV représente un copolymère séquencé ou un copolymère aléatoire.

6. Composition selon la revendication 1, dans laquelle le composé conjugué absorbant les UV comprend un composé de type (U-X)n-C-(X-U1)m,
dans lequel, U représente un fragment de composé absorbant les UV, U1 représente un fragment de composé absorbant les UV qui présente une structure chimique différente de celle de U, X représente un groupe thioéther, un groupe amine, un groupe amide, un groupe ester, un groupe uréthane, un groupe sulfonamide, un groupe -CO-NH-NH-CO-, C représente un fragment de composé conjugué et n et m représentent chacun un nombre entier et dans lequel n et m sont ≥ 1.

7. Composition selon la revendication 1, dans laquelle le composé conjugué absorbant les UV comprend, 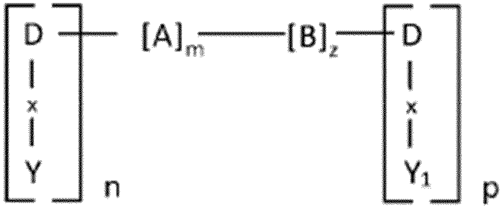 dans laquelle Y représente un fragment de composé absorbant les UV, Y₁ représente un fragment de composé absorbant les UV qui présente une structure chimique différente de celle de Y, X représente un groupe thioéther, un groupe amine, un groupe amide, un groupe ester, un groupe uréthane, un groupe sulfonamide, un groupe -CO-NH-NH-CO-, D représente un résidu d'un groupe vinyle, acrylate, méthacrylate ou acrylamide, A représente un résidu d'un monomère de type vinyle, acrylate, méthacrylate ou acrylamide, B représente un résidu d'un monomère de type vinyle, acrylate, méthacrylate ou acrylamide qui est différent de A, n et p représentent chacun un nombre entier et dans lequel n et p sont ≥ 1 et m et z représentent chacun un nombre entier et dans lequel m est ≥ 0, et z est ≥ 0. La structure de formule (4) peut être un copolymère séquencé ou un copolymère aléatoire.

8. Composition selon la revendication 7 dans laquelle le composé conjugué absorbant les UV représente un copolymère séquencé ou un copolymère aléatoire.

9. Composition selon la revendication 1, dans laquelle les composés conjugués absorbant les UV comprennent des composés à base de benzophénone, des composés à base de benzotriazole ou des composés à base de benzimidazole dont l'absorbance est comprise entre environ 200 et environ 380 nm.

10. Composition selon la revendication 9, dans laquelle les composés conjugués absorbant les UV comprennent un ou plusieurs composés à base de benzotriazole absorbant les UV comprenant les résidus de réaction d'un groupe acrylamide, d'un groupe acrylate, d'un groupe méthacrylate, d'un groupe maléimide, de groupes acrylonitrile ou d'un groupe vinylsulfone.

11. Composition de formulation topique selon la revendication 1, formulée sous la forme d'une formulation d'écran solaire comprenant un écran solaire à usage quotidien, un écran solaire résistant à l'eau, ou des combinaisons de ceux-ci ; une formulation cosmétique comprenant une crème hydratante, un fond de teint, un rouge à lèvres, un brillant à lèvres, un baume à lèvres, un anticerne, un surligneur, un fard à joues, des ombres à paupières, des nettoyants, des toniques, des sérums, des produits anti-âge, des sprays fixants ou des combinaisons de ceux-ci ; une composition de produit capillaire comprenant un shampoing, un après-shampoing, un après-shampoing sans rinçage, une mousse capillaire, un gel capillaire, une laque capillaire, une crème bouclante, une cire capillaire, une huile de traitement, un soin capillaire médicamenteux ou des combinaisons de ceux-ci.
